# EUROPÄISCHE PATENTSCHRIFT

(11) **EP 0 606 683 B1**
(45) Veröffentlichungstag und Bekanntmachung des Hinweises auf die Patenterteilung: **02.01.2003**
(21) Anmeldenummer: 93250286.7
(22) Anmeldetag: 22.10.1993
(51) Int. Cl.: C07K 14/575, A61K 49/00, A61K 51/08

(54) **Neue Mittel zur Diagnose von Gefässerkrankungen**
Means for diagnosis of angiogenic diseases
Agents pour la diagnose des maladies vasculaires

(30) Priorität: 13.01.1993 DE 4301871
(43) Veröffentlichungstag der Anmeldung: 20.07.1994
(73) Patentinhaber: INSTITUT FÜR DIAGNOSTIKFORSCHUNG GmbH AN DER FREIEN UNIVERSITÄT BERLIN, 14050 Berlin (DE)
(72) Erfinder: Dinkelborg, Ludger, Dr., D-13595 Berlin (DE); Erber, Sebastian, Dr., D-13505 Berlin (DE); Hilger, Christoph Stephan, Dr., D-13353 Berlin (DE); Kramp, Wolfgang, Dr., D-13503 Berlin (DE); Schier, Hans-Martin, Dr., D-10625 Berlin (DE); Speck, Ulrich, Dr., D-13465 Berlin (DE); Gries, Heinz, Dr., D-10717 Berlin (DE); Platzek, Johannes, Dr., D-14195 Berlin (DE); Reiser, Joseph H., Mendham NJ 07945 (US)
(74) Vertreter: Mager, Knut

(56) Entgegenhaltungen:
- EP-A- 0 402 313
- WO-A-91/13089
- WO-A-91/16919
- WO-A-93/12819
- J.NUCL.MED., Bd. 33, Nr. 5, Mai 1992 Seite 845 C.KURATA ET AL 'Localisation of radioiodinated ET-1 in injured aortic tissue of cholesterol fed rabbits'
- BR.J.PHARMACOL., Bd. 107, 1992 Seiten 637-639, P.MOLENAAR ET AL 'Characterisation of two new ET-B selective radioligands,...'
- LIFE SCIENCES, Bd. 50, Nr. 25, 1992 Seiten 2001-2009, B-D.LÖFFLER ET AL 'A rabbit antiserum raised against the hexapeptide ET(16-21) shows different binding affinities for ET-1,-2 and -3'
- EUR.J.PHARMACOL., Bd. 118, Nr. 2/3, 6.Juni 1991 Seiten 165-169, C.TAKASAKI ET AL 'Structure-activity relationships of saratoxins'
- J.MED.CHEM., Bd. 35, Nr. 9, 1.Mai 1992 Seiten 1493-1508, A.M.DOHERTY 'Endothelin: a new challenge'
- PRAT E.A. EUR.J.NUCL.MED. Bd. 20, Nr. 12, Dezember 1993, Seiten 1141 - 1145

## Beschreibung

Die Erfindung betrifft den in den Patentansprüchen gekennzeichneten Gegenstand, d. h. Metallkomplex-Konjugate von Endothelinen, Endothelinderivaten, Teilsequenzen von Endothelinen, Endothelin-Analoga oder Endothelin-Antagonisten, sowie diese Verbindungen enthaltende Mittel, ihre Verwendung zur Herstellung von Diagnostika, sowie Verfahren zur Herstellung dieser Verbindungen und Mittel. Dabei werden *in vivo*-applizierbare Metallkomplex-Konjugate von Endothelinen, Endothelinderivaten, Teilsequenzen von Endothelinen, Endothelin-Analoga oder Endochelin-Antagonisten, vorzugsweise zur nicht-invasiven Bildgebung von Gefäßerkrankungen eingesetzt.

Die Atherosklerose ist eine chronische, fortschreitende Erkrankung der Blutgefäße, die bisher Klinisch erst in ihrem Spätstadium diagnostiziert werden kann. Atheroskierotische Gefäßveränderungen werden heute konventionell durch die Arteriographie dargestellt. Hierzu wird über einen Katheter ein Kontrastmittel in das jeweilige interessierende Gefäß appliziert und mittels Röntgenstrahlen solche Gefäßbereiche detektiert, die eine Verengung aufweisen. Ein Nachteil dieser Methode liegt darin, daß nur Teilbereiche des Gefäßsystems betrachtet werden können. Da die Arteriographie eine invasive Methode ist, können bei ihrer Anwendung Komplikationen wie z. B. Schmerzen, Perforationen der Arterien, Arrhythmien, Herz- oder Himinfarkte auftreten, die unter ungünstigen Umständen sogar den Tod des Patienten zur Folge haben können.

Weiterhin werden Verfahren, die auf dem Einsatz von Ultraschall beruhen, sowie die MR-Tomographie zur Diagnose der Atherosklerose eingesetzt.

Alle derzeit angewendeten Verfahren beinhalten jedoch den großen Nachteil, daß sie atherosklerotische Gefäßveränderungen durch den an diesen Stellen verminderten Blutfluß oder grobe Arterienwandveränderungen detektieren und daher nur späte Stadien der Atherogenese aufzeigen können.

Eine frühe Erkennung der Atherosklerose wäre z. B. für die Kontrolle des Therapieerfolges von Diät, Kalziumantagonisten, Lipid- und Bluthochdrucksenkern, der Kontrolle der Restenose nach Angioplastie und der Diagnose koronarer Herzerkrankungen sowie der Detektion von thrombotischen Gefäßablagerungen von großer Bedeutung.

Nicht-invasive Techniken zur Diagnose der Atherosklerose wurden beschrieben. So wur den mit Radioisotopen markierte Antikörper oder auch markierte "Low Densiry Lipoproteine" (LDL) vorgestellt, die an atherosklerotische Wandbereiche binden (Lees et al. 1983, J. Nucl. Med. 24, 154-156; Kaliman et al. 1985, Circulation, 72, 300: Virgolini et al 1991, Eur. J. Nucl. Med., 18, 944-947). Diese Methoden beinhalten jedoch entscheidende Nachteile wie z.B. die antigene Wirkung der Antikörper auf den Organismus und die lange Zeitdauer (mehrereTage), die zur Isolierung, Reinigung und Markierung des LDL aus dem Blut des Patienten benötigt wird. Vor allem aber zeigen diese großen Moleküle eine lange Halbwertszeit im Blut, die zusammen mit einer hohen Hintergrundstrahlung im gesamten Körper, eine Lokalisation der atherosklerotischen Läsionen erschwert, wenn nicht gar unmöglich macht.

Shih et al. (1990, Proc. Natl. Acad. Sci., 87, 1436-1440) synthetisierten Teilsequenzen des LDL-Proteinanteils (apo-B-100), die zwar noch an die atherosklerotischen Plaques binden, aber sich durch eine weitaus kürzere Halbwertszeit im Blut und ein verbessertes Signal-Rausch-Verhältnis hervorheben. Aufgrund einer zu geringen Affinität zum Plaque und/oder der geringen Dichte der Bindungsstellen dieser Peptide im Plaque, konnte auch mit diesen apo-B-Peptiden keine erfolgreiche *in vivo* Diagnose der Atherosklerose etabliert werden. P. Molenaar et al. beschreiben in Br. J. Pharmacol. (1992), Vol. 107, S. 637-639 zwei neue, radioaktiv markierte Endothelin-Rezeptor-Liganden sowie Experimente, welche die Bindungsaffinität dieser Liganden nachweisen.
In WO 91/16919 werden synthetische Peptide beschrieben, die von Apolipoprotein B, Apolipoprotein A-I oder Elastin abgeleitet sind. Die Peptide sollen sich bevorzugt in atherosklerotischen Läsionen anreichern.

Der Erfindung liegt daher die Aufgabe zugrunde, neue Verbindungen und Mittel zur Verfügung zu stellen, die für ein neues, nicht-invasives Verfahren zur Diagnose, insbesondere der Diagnose der frühen, noch nicht stenotischen Gefäßerkrankungen geeignet sind. Diese Aufgabe wird durch die erfindungsgemäßen Metallkomplex-Konjugate von Endothelinen, Endothelinderivaten, Teilsequenzen von Endothelinen, Endothelin-Analoga oder Endothelin-Antagonisten erfüllt.

Endotheline sind physiologisch aktive Peptide, die sowohl hormonelle als auch neuroregulatorische Funktionen im Organismus ausüben (MacCumber et al. 1989, Proc. Natl. Acad. Sci., 86, 7285-7289; Yanagisawa et al. 1989, Trends Pharmacol. Sci., 10, 374-378; LeMonnier de Gouville et al., 1989, Life Sci., 45, 1499-1513; Yanagisawa er al., 1988, Nature, 332, 411-415). Vier verschiedene Isotypen konnten bisher beim Menschen nachgewiesen werden (Inoue et al., 1989, Proc. Natl. Acad. Sci., 86, 2863-2867). Endothelin 1 ist ein Polypeptid mit der folgenden Sequenz aus 21 Aminosäuren: 
Cys-Ser-Cys-Ser-Ser-Leu-Met-Asp-Lys-Glu-Cys-Val-Tyr-Phe-Cys-His-Leu-Asp-Ile-Ile-
Trp
 (Yanagisawa et al., 1988, Nature, 332, 411-415). Vom Gefäßendothel wird eine inaktive Vorstufe des Endothelins, das Big Endothelin gebildet. Nach Abspaltung eines Heptadecapeptids durch das Endothelin-Converting-Enzyme (ECE) entsteht Endothelin, das an spezifische Rezeptoren der glatten Gefäßmuskulatur bindet. Dort führt es zu einer Ca⁺⁺- vermittelten Kontraktion der glatten Muskelzellen (Yanagisawa et al., 1988. Nature, 332, 411-415; Takuwa et al. 1991, Contrib. Nephrol., 90, 99-104).

Eine der frühen irreversiblen Veränderungen während der Atherogenese ist die u. a. durch Wachstumsfaktoren (z.B. PDGF) hervorgerufene Proliferation glatter Muskelzellen der Gefäßwand (Desmoulière und Gabbiani 1992, Cerebrovasc. Dis., 2, 63-71: Ross 1986, N. Engl. J. Med., 314, 448-501). Diese proliferierenden Zellen verändern ihre Morphologie sowie ihre physiologische Funktion (Desmoulière und Gabbiani 1992, Cerebrovasc. Dis., 2, 63-71). Durch die *in vitro*-Inkubation von humanen atheromatösen Koronararterien mit ¹²⁵I-Endothelin 1 konnte gezeigt werden, daß eine vermehrte Bindung von ¹²⁵I-Endothelin 1 im Bereich der Tunica media sowie in Bereichen der Vasa vasora auftritt (Dashwood et al., 1991, J. Cardiovasc. Pharmacol., 17, S458-S462). Durch die Applikation von ¹²⁵I-Endothelin 1 in Kaninchen, deren abdominale Aorta zuvor mittels Ballonkatheter deendothelisiert wurde, konnte eine vermehrte Aufnahme von ¹²⁵I-Endothelin 1 in den deendothelisierten Bereichen der Aorta gezeigt werden, die auf eine höhere Dichte der Bindungsstellen für Endothelin 1 in diesen verletzten Gefäßregionen schließen läßt (Kurata et al. 1992, J. Nucl. Med., 33, 845). Diese Untersuchungen lassen darauf schließen, daß die proliferierenden glatten Muskelzellen auch weiterhin den Endothelin-Rezeptor exprimieren.

Endothelin 1 übt eine starke vasokonstriktorische Wirkung auf die glatte Gefäßmuskulatur aus (A. M. Doherty, 1992, Medicinal Chemistry, 35, 1493-1508). Daher können nur relativ niedrige Konzentrationen dem Organismus i. v. appliziert werden. Höhere Konzentrationen können von Teilsequenzen der Endotheline, Endothelinderivate, Endothelin-Analoga bzw. Endothelin-Antagonisten, die zwar noch an den Endothelin-Rezeptor binden, nicht aber zu einer so ausgeprägten Kontraktion der glatten Muskelzellen führen, appliziert werden.

Da Endotheline sehr schnell über die Nieren ausgeschieden werden, ist die durch die Aufnahme der Endotheline in anderen Organen oder Geweben bedingte störende Hintergrundstrahlung äußerst gering.

Es wurde gefunden, daß radioaktiv markierte Endotheline, Endothelinderivate, Teilsequenzen von Endothelinen sowie geeignete Endothelin-Antagonisten und Endothelin-Analoga sich in atherosklerotischen Gefäßläsionen überraschend stark anreichern und somit eine für die Darstellung mit einer Szintillationskamera oder anderen geeigneten, in der Nuklearmedizin angewandten Apparaturen ausreichende Konzentration erreichen. Überraschend war auch der Befund, daß die erfindungsgemäß eingesetzten Substanzen diese Konzentration *in vivo* so rasch erreichen und die Bindung so stabil ist, daß nach Abtransport und Ausscheidung der überschüssigen Endothelinderivate, Antagonisten oder sonstigen an den Rezeptor bindende Substanzen eine hohe, für die Diagnostik ausreichende Konzentration verbleibt. Weiterhin war überraschend, daß die Anreicherung ganz bevorzugt an den zu diagnostizierenden, auf unterschiedlichste Weise veränderten Arterienwandbereichen erfolgt, obwohl Endothelin an allen Gefäßbezirken wirkt.

Weiterhin sind die erfindungsgemäß eingesetzten Substanzen besonders geeignet, da sie im Gegensatz zu vielen anderen geprüften Substanzklassen und Substanzen sich nicht zusätzlich und unspezifisch in anderen Geweben und Organen anreichern, was für eine Eignung als Diagnostikum entscheidend ist.

Die erfindungsgemäßen Metallkomplex-Konjugate von Endothelinen, Endothelinderivaten, Teilsequenzen von Endothelinen, Endothelin-Analoga oder Endothelin-Antagonisten, sowie die aus ihnen bereiteten Lösungen erfüllen die genannten Anforderungen in überraschend hohem Maße. Sie besitzen sowohl eine höhere Anreicherung in pathologischen Gefäßbereichen als auch eine durch die günstigere Pharmakokinetik bedingte bessere Kontrasteigenschaft, als die bisher für die Erfassung von Gefäßerkrankungen beschriebenen Diagnostika. Die praktische Anwendung der neuen erfindungsgemäßen Substanzen wird auch durch deren hohe chemische Stabilität erleichtert.

Die erfindungsgemäßen Komplexe von Verbindungen der allgemeine Formel I

E-L-(K)_{b} (I)

mit Metallionen der Ordnungszahlen 21-32, 37-39, 42-51 und 57-83,
sind dadurch gekennzeichnet,
daß
- E: für einen Rest, abgeleitet von Endothelinen, Endothelinderivaten, Endothelin-Teilsequenzen, Endothelin-Analoga oder Endothelin-Antagonisten steht,
und wobei der Rest E die Eigenschaft aufweist, selektiv an Endothelin-Rezeptoren zu binden,
- L: eine direkte Bindung oder einen Rest Z₁-R-Z₂ darstellt,
worin
R für einen gegebenenfalls durch ein oder mehrere Sauerstoff- und/oder Schwefelatome und/oder Carbonyl, -NHCO-, -N(C₁₋₆Alkyl)CO-, -NHund -N(C₁₋₆Alkyl)-Reste unterbrochenen und gegebenenfalls durch Hydroxy-und/oder Epoxygruppen substituierten geradkettigen, verzweigten, gesättigren oder ungesättigten C₁₋₂₀-Alkylrest steht,
Z₁ und Z₂ unabhängig voneinander eine Gruppe -O-, -S-, -(C=O)O-, -NH-(C=S)NH-, -(C=O)-, -(C=S)O-, -NH-, -NH-(C=O)- oder -NH-(C=S)-ist,
oder für einen Rest der Formel α 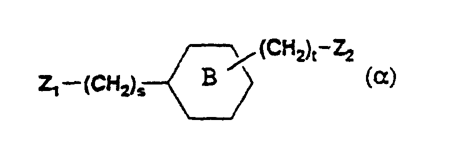 steht, worin
s und t voneinander unabhängig für die Ziffern 0, 1, 2 oder 3 stehen, der Ring B einen Phenyl- oder Cyclohexylrest bedeutet und Z₁ und Z₂ die vorstehend angegebene Bedeutung haben,
- b: für die Ziffer 1 steht,
- K: einen Chelatbildnerrest der allgemeinen Formel II A oder II B 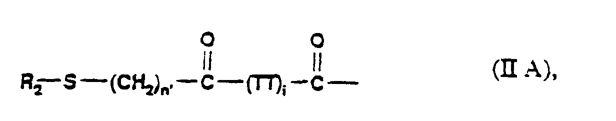 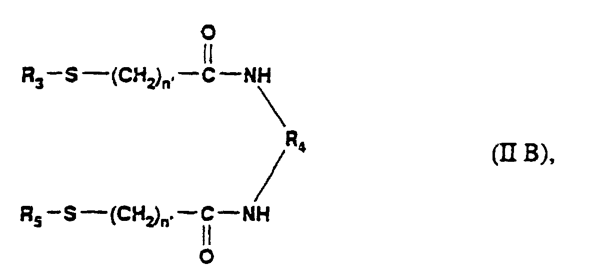
darstellt,
worin
R₂, R₃ und R₅ unabhängig voneinander für ein Wasserstoffatom, einen gegebenenfalls mit einer Hydroxylgruppe, einem C₁₋₄-Alkoxy-, einem Carboxyl- oder einem Sulfonsäurerest substituierten (C₁₋₆-Alkyl)CO-, (C₆₋₈-Aryl)CO- oder (C₇₋₉-Arylalkyl)CO-Rest stehen und
R₄ für einen Rest der Formel II C oder II D 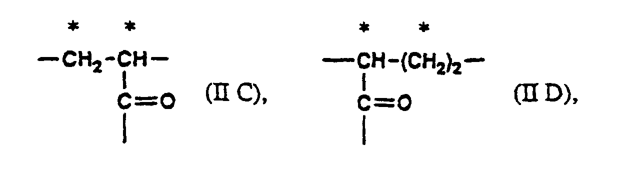 worin
die mit einem * gekennzeichneten Kohlenstoffatome an die Iminogruppen der Formel II B gebunden sind und
n' für die Ziffern 1 oder 2,
i für eine beliebige Zahl von 2 bis 6, und
TT für α- und/oder β- Aminosäuren, welche in üblicher Weise über Amidgruppen verbunden sind,
stehen,
sowie Chelatbildnerreste, die sich von Aminopolycarbonsäuren der Formel II K oder II L 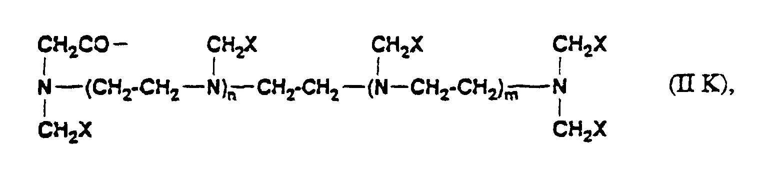 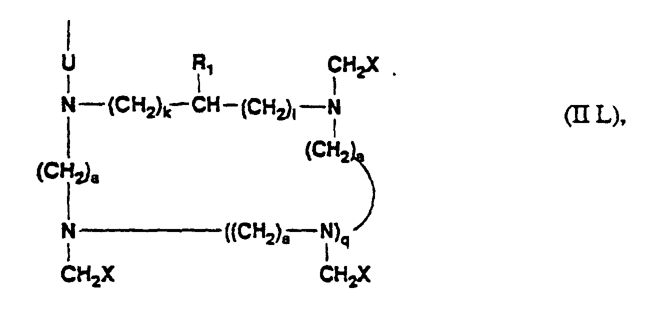 ableiten, wobei
n und m jeweils für die Ziffern 0, 1, 2, 3 oder 4 stehen, wobei n und m nicht mehr als 4 ergeben,
a für die Ziffern 2, 3, 4, oder 5
k für die Ziffern 1, 2, 3, 4, oder 5,
l für die Ziffern 0, 1, 2, 3, 4, oder 5 und
q für die Ziffern 0, 1 oder 2 stehen,
U ein Wasserstoffatom oder einen C₁₋₆-Alkylrest darstellt, welcher gegebenenfalls mit einer oder mehreren Hydroxy-Gruppen substituiert ist und eine Bindung zu L enthält,
die Reste X unabhängig voneinander jeweils Wasserstoffatome, COOH-Gruppen, Estergruppen oder Amidgruppen mit 1-6 Kohlenstoffatomen in dem Alkylrest bedeuten,
R₁ für eine Bindung zu L oder ein Wasserstoffatom steht,
sowie Chelatbildnerreste der Formel II M,

Cp(aa)Cp- (II M)

worin
Cp für ein geschütztes Cystein und (aa) für eine der natürlich vorkommenden Aminosäuren steht,
sowie cysteinreiche Aminosäuresequenzen der Metallothionine -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- und analoge Sequenzen, in denen Serin durch Threonin, Glycin oder Alanin ersetzt ist.

Die in den erfindungsgemäßen Komplexen enthaltenen Metallionen weisen Eigenschaften auf, die es gestatten, diese Metallionen mittels physikalischer Methoden nachzuweisen. Die oben genannten Eigenschaften sind beispielsweise der Paramagnetismus, die Radioaktivität unter Aussendung von α-, β- und γ-Strahlung oder ein großer Absorptionsquerschnitt für Röntgen- oder andere Strahlen. Insbesondere weisen die Übergangselemente, die Lanthaniden und die Hauptgruppenelemente der 4, bis 6, Periode diese Eigenschaften auf.

Besonders bevorzugt sind Ionen der Isotope der Metalle Tc, Re, In, Cr, Mn, Fe, Co, Ni, Cu, Pr, Nd, Sm, Y, Gd, Tb, Dy, Ho, Er und La.

Die erfindungsgemäßen Komplexe von Verbindungen der allgemeinen Formel (I)

E-L-(K)_{b} (I)

mit Metallionen der Ordnungszahlen 21-32, 37-39, 42-51 und 57-83,
enthalten ferner Reste E, welche sich von Endothelinen, Endothelinderivaten, Endothelin-Teilsequenzen, Endothelin-Analoga oder Endothelin-Antagonisten ableiten. Diese Reste E weisen die Eigenschaft auf, selektiv an Endothelin-Rezeptoren zu binden.

Die Reste E leiten sich bevorzugt von Endothelinen ab.

Von den Endothelinen ist das Endothelin 1 besonders bevorzugt, welches die folgende Aminosäuresequenz oder Teile davon aufweist:

Cys-Ser-Cys-Ser-Ser-Leu-Met-Asp-Lys-Glu-Cys-Val-Tyr-Phe-Cys-His-Leu-Asp-Ile-
Ile-Trp.

Die hydrophobe Region des Endothelin 1, His¹⁶-Trp²¹ ist für die Bindung an den Rezeptor essentiell (Kimura et al., 1988, Biochem. Biophys. Res. Comm., 156, 1182-1186).

Weiterhin bevorzugt sind Reste E, welche sich von Endothelin-Teilsequenzen ableiten.

Besonders bevorzugte Teilsequenzen der erfindungsgemäß eingesetzten Endotheline sind daher solche Peptide, welche die Aminosäuresequenz
-His-Leu-Asp-Ile-Ile-Trp- aufweisen.

Ferner sind Reste E bevorzugt, die sich von Endothelin-Analoga ableiten.

Besonders bevorzugte erfindungsgemäße Endothelin-Analoga weisen beispielsweise die folgenden Aminosäuresequenzen oder Teile davon auf:

-Cys-Ser-Cys-Ser-Ser-Trp-Leu-Asp-Lys-Glu-Cys-Val-Tyr-Phe-Cys-His-Leu-Asp-Ile-Ile-
Trp- (Endothelin 2).

-Cys-Thr-Cys-Phe-Thr-Tyr-Lys-Asp-Lys-Glu-Cys-Val-Tyr-Tyr-Cys-His-Leu-Asp-Ile-Ile-
Trp- (Endothelin 3).

-Cys-Ser-Ala-Ser-Ser-Leu-Met-Asp-Lys-Glu-Ala-Val-Tyr-Phe-Cys-His-Leu-Asp-Ile-Ile-
Trp- (Ala-Endothelin).

-Cys-Ser-Cys-Asn-Ser-Trp-Leu-Asp-Lys-Glu-Cys-Val-Tyr-Phe-Cys-His-Leu-Asp-Ile-Ile-
Trp- (VIC).

-Cys-Ser-Cys-Lys-Asp-Met-Thr-Asp-Lys-Glu-Cys-Leu-Asn-Phe-Cys-His-Gln-Asp-Val-
Ile-Trp- (Sarafotoxin S 6a).

-Ala-Ser-Cys-Ser-Ser-Leu-Met-Asp-Lys-Glu-Cys-Val-Tyr-Phe-Ala-His-Leu-Asp-Ile-Ile-
Trp-.

-Ala-Ser-Ala-Ser-Ser-Leu-Met-Asp-Lys-Glu-Ala-Val-Tyr-Phe-Ala-His-Leu-Asp-Ile-Ile-
Trp-.

-Cys-Ser-Cys-Ser-Ser-Trp-Leu-Asp-Lys-Glu-Ala-Val-Tyr-Phe-Ala-His-Leu-Asp-Ile-Ile-
Trp-.

Desweiteren bevorzugt sind Reste E, welche sich von Endothelin-Antagonisten ableiten.

Bevorzugt eingesetzte Endothelin-Antagonisten sind zyklische Pentapeptide wie beispielsweise: -D Trp-D Asp-Pro-D Val-Leu- oder

-D Glu-Ala-allo D Ile-Leu-D Trp-.

Ferner sind in den erfindungsgemäßen Komplexen von Verbindungen der allgemeinen Formel (I)

E-L-(K)_{b} (I)

mit Metallionen der Ordnungszahlen 21-32, 37-39, 42-51 und 57-83,

Reste L enthalten, welche die Komplexbildnerreste K, die ihrerseits die jeweiligen Metallionen binden, mit den Resten E, abgeleitet von Endothelinen, Endothelinderivaten, Endothelin-Teilsequenzen, Endothelin-Analoga oder Endothelin-Antagonisten, verbinden.

Bevorzugte erfindungsgemäßeKomplexe enthalten als Rest L einen geradkettigen, verzweigten, zyklischen, aliphatischen, aromatischen oder arylaliphatischen Alkylenrest mit bis zu 20 Kohlenstoffatomen.

Bevorzugt sind ferner erfindungsgemäße Komplexe in denen L für Z₁-R-Z₂ steht,
worin
Z₁ und Z₂ unabhängig voneinander eine Gruppe -(C=O)O-, -(C=O)-, -NH-, -NH-(C=O)- bedeuten und
R eine geradkettige Mono- bis Dekamethylengruppe darstellt
oder einen Rest der Formel α 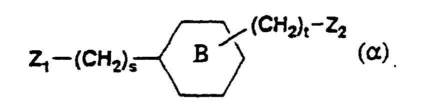 bedeutet, worin
s gleich 1 ist und t gleich 0 bedeutet und für den Ring B Phenylen und
Z₁ und Z₂ unabhängig voneinander für eine Gruppe -NH-(C=S)-, -NH-(C=S)NH-, -(C=O)O-, -(C=O)-, -NH-, -NH-(C=O)- stehen.

Die Komplexbildnerreste K, welche ebenfalls in den erfindungsgemäßen Komplexen enthalten sind, müssen in der Lage sein, die jeweiligen Metallionen in Form von koordinativen und/oder kovalenten Bindungen zu binden.

Je nach Art des zu bindenden Metallions, in Abhängigkeit von deren Ladung, Oxidationszahl und Atom- bzw. Ionenradius muß der Komplexbildnerrest K derart gewählt werden, daß eine effektive und für die erfindungsgemäße Verwendung der erfindungsgemäßen Komplexe ausreichende Bindung erzeugt wird.

Bevorzugte Komplexbildnerreste K sind
S-Benzoylthioacetylglycylglycylglycin, N,N'-Bis(benzoylthioacetyl)-2,3-diaminopropionsäure, N,N'-Bis(benzoylthioacetyl)-3,4-diaminobuttersäure, N,N'-Bis(benzoylthioacetyl)-4,5-diaminopentansäure,
Cys(Acm)GlyCys(Acm)GlyGlyArgGlyAspSer, Ethylendiamintetraessigsäure, Diethylentriaminpentaessigsäure, trans-1,2-Cyclohexandiamintetraessigsäure, 1,4,7,10-Tetraazacyclododecantetraessigsäure, 1,4,7-Triazacyclononan-triessigsäure, 1,4,8,11-Tetraazatetradecantetraessigsäure, 1,5,9-Triazacyclododecantriessigsäure, 1,4,7,10-Tetraazacyclododecantriessigsäure und 3,6,9,15-Tetraazabicyclo[9,3,15]-pentadeca-1(15),11,13-trien-triessigsäure. Gewünschtenfalls kann ein Teil der Carbonsäuren als Ester und/oder Amid vorliegen.

Sollen die Carbonsäuregruppen zumindest teilweise als Amide vorliegen, so sind tertiäre Amide bevorzugt. Als Reste kommen gesättigte, ungesättigte, gerad- oder verzweigtkettige oder zyklische Kohlenwasserstoffe mit bis zu 5 C-Atomen in Frage, die gegebenenfalls durch 1 bis 3 Hydroxy- oder C₁-C₄-Alkoxy-Gruppensubstituiert sein können. Beispielsweise genannt seien die Methyl-, Ethyl-, 2-Hydroxyethyl-, 2-Hydroxy-1-(hydroxymethyl)-ethyl-, 1-(Hydroxymethyl)-ethyl, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethyl-Gruppe. Der Amidrest kann auch ein unter Einschluß des Amid-Stickstoffs gebildeter heterozyklischer 5- oder 6-Ring sein. Beispielhaft genannt seien: der Pyrrolidinyl-, Piperidinyl-, Pyrazolidinyl-, Pyrazolinyl-, Piperazinyl-, Morpholinyl-, Imidazolidinyl-, Oxazolidinyl-, Thiazolidinyl-Ring.

Besonders bevorzugte Ausführungsbeispiele der Komplexe von Verbindungen der allgemeinen Formel (I)

E-L-(K)_{b} (I)

mit Metallionen der Ordnungszahlen 21-32, 37-39, 42-51 und 57-83,
sind beispielsweise
a) ^{99m}Tc-Komplex von S-Benzoylthioacetyl-Gly-Gly-Gly-Gly-Asp-His-Leu-Asp-Ile-Ile-Trp,
b) 
⁹⁹ᵐTc-Komplex von N,N´-Bis(S-benzoylthioacetyl)-3,4-diaminobutyryl-Cys-Ser-Cys-
Ser-Ser-Leu-Met-Asp-Lys-Glu-Cys-Val-Tyr-Phe-Cys-His-Leu-Asp-Ile-Ile-Trp,
c) 
99mTc-Komplex von N,N´-Bis(S-benzoylthioacetyl)-4,5-diamino-1-oxo-pentyl-Cys-Ser-
Cys-Ser-Ser-Leu-Met-Asp-Lys-Glu-Cys-Val-Tyr-Phe-Cys-His-Leu-Asp-Ile-Ile-Trp,
d) ^{99m}Tc-Komplex von N-[3,6,9-Triaza-1-oxo-3,6,9,9-tetra-(hydroxycarbonylmethyl)-nonyl]-Gly-His-Leu-Asp-Ile-Ile-Trp,
e) ¹¹¹In-Komplex von N-[3,6,9-Triaza-1-oxo-3,6,9,9-tetra-(hydroxycarbonylmethyl)-nonyl]-Gly-His-Leu-Asp-Ile-Ile-Trp.
f) Gd(III)-Komplex von N-[3,6,9-Triaza-1-oxo-3,6,9,9-tetra-(hydroxycarbonylmethyl)-nonyl]-Gly-His-Leu-Asp-Ile-Ile-Trp, Natriumsalz,
g ) Gd(III)-Komplex von 1-{2-Hydroxy-3-[4-(Gly-His-Leu-Asp-Ile-Ile-Trp-thiouridyl)phenoxy]propyl}1,4,7,10-tetraaza-4,7,10-tris-(carboxylatomethyl)-cyclododecan,
h) 
⁹⁹ᵐTc-Komplex von Cys(Acm)-Gly-Cys(Acm)-Cys-Ser-Cys-Ser-Ser-Leu-Met-Asp-
Lys-Glu-Cys-Val-Tyr-Phe-Cys-His-Leu-Asp-Ile-Ile-Trp,
i) ^{99m}Tc-Komplex von Cyclo(Trp-Leu-Val-Pro-Asp)-Cys(Acm)-Gly-Cys(Acm),
k) 
99mTc-Komplex von 4-Mercaptobutyrimidyl-Cys-Ser-Ala-Ser-Ser-Leu-Met-Asp-Lys-
Glu-Ala-Val-Tyr-Phe-Cys-His-Leu-Asp-Ile-Ile-Trp,
l) ^{99m}Tc-Komplex von 3-Thiopropionyl-Gly-Asp-His-Leu-Asp-Ile-Ile-Trp,
m) ^{99m}Tc-Komplex von 2-(Acetylthio)succinyl-Gly-Asp-His-Leu-Asp-Ile-Ile-Trp

Gegenstand der vorliegenden Erfindung sind ferner neue Konjugate zwischen dem Komplexbildnerrest K und dem Rest E, abgeleitet von Endothelinen, Endothelinderivaten, Endothelin-Teilsequenzen, Endothelin-Analoga oder Endothelin-Antagonisten, welche über den Rest L miteinander verknüft sind, der allgemeinen Formel (I)

E-L-(K)_{b} (I)

worin
- E: für einen Rest, abgeleitet von Endothelinen, Endothelinderivaten, Endothelin-Teilsequenzen, Endothelin-Analoga oder Endothelin-Antagonisten, steht,
und wobei der Rest E die Eigenschaft aufweist, selektiv an Endothelin-Rezeptoren zu binden,
- L: eine direkte Bindung oder einen Rest Z₁-R-Z₂ darstellt,
worin
R für einen gegebenenfalls durch ein oder mehrere Sauerstoff- und/oder Schwefelatome und/oder Carbonyl, -NHCO-, -N(C₁₋₆Alkyl)CO-, -NHund -N(C₁₋₆-Alkyl)-Reste unterbrochenen und gegebenenfalls durch Hydroxy-und/oder Epoxygruppen substituierten geradkettigen, verzweigten, gesättigten oder ungesättigten C₁₋₂₀ Alkylrest steht,
Z₁ und Z₂ unabhängig voneinander eine Gruppe O-, -S-, -(C=O)O-, -NH-(C=S)NH-, -(C=O)-, -(C=S)O-, -NH-, -NH-(C=O)- oder -NH-(C=S)-sind,
oder für einen Rest der Formel α 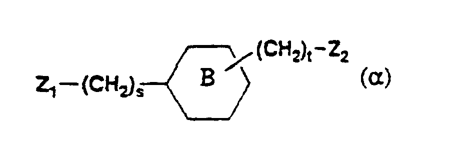 steht, worin
s und t voneinander unabhängig für die Ziffern 0, 1, 2 oder 3 stehen, der Ring B einen Phenyl- oder Cyclohexylrest bedeutet und Z₁ und Z₂ die vorstehend angegebene Bedeutung haben,
- b: für die Ziffer 1 steht,
- K: einen Chelatbildnerrest der allgemeinen Formel II A oder II B 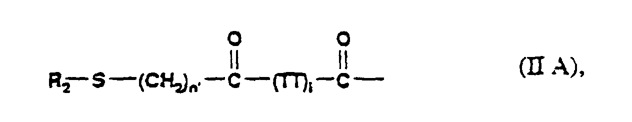 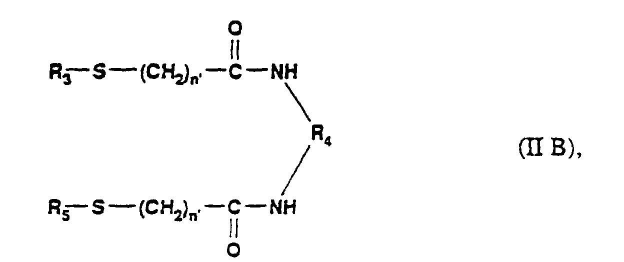
darstellt, worin
R₂, R₃ und R₅ unabhängig voneinander für ein Wasserstoffatom, einen gegebenenfalls mit einer Hydroxylgruppe, einem C₁₋₄-Alkoxy-, einem Carboxyl- oder einem Sulfonsäurerest substituierten (C₁₋₆-Alkyl)CO-, (C₆₋₈-Aryl)CO- oder (C₇₋₉-Arylalkyl)CO-Rest stehen und
R₄ für einen Rest der Formel II C oder II D 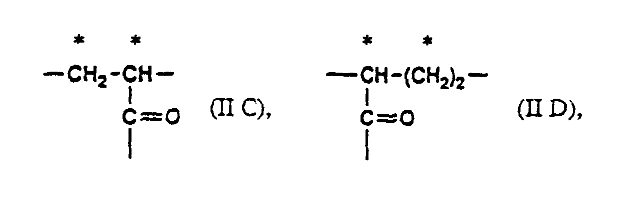 worin
die mit einem * gekennzeichneten Kohlenstoffatome an die Iminogruppen der Formel II B gebunden sind und
- n': für die Ziffern 1 oder 2,
- i: für eine beliebige Zahl von 2 bis 6, und
- TT: für α- und/oder β- Aminosäuren, welche in üblicher Weise über Amidgruppen verbunden sind,
stehen,
sowie Chelatbildnerreste, die sich von Aminopolycarbonsäuren der Formel II K oder II L 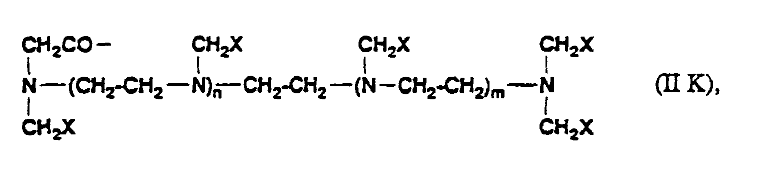 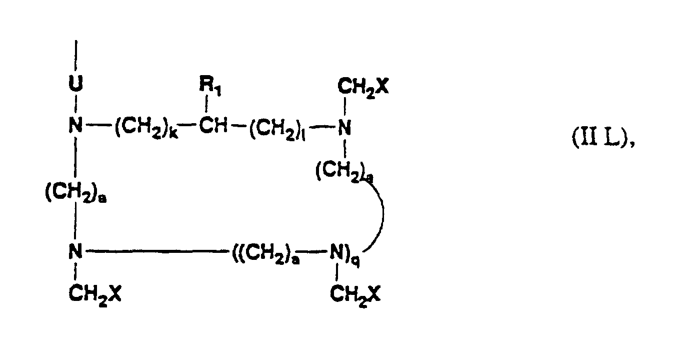 ableiten, wobei
- n und m: jeweils für die Ziffern 0, 1, 2, oder 4 stehen, wobei n und m nicht mehr als 4 ergeben,
- a: für die Ziffern 2, 3, 4, oder 5
- k: für die Ziffern 1, 2, 3, 4, oder 5,
- l: für die Ziffern 0, 1, 2, 3, 4, oder 5 und
- q: für die Ziffern 0, 1 oder 2 stehen,

U ein Wasserstoffatom oder einen C₁₋₆-Alkylrest darstellt, welcher gegebenenfalls mit einer oder mehreren Hydroxy-Gruppen substituiert ist und eine Bindung zu L enthält,
die Reste X unabhängig voneinander jeweils Wasserstoffatome, COOH-Gruppen, Estergruppen oder Amidgruppen mit 1-6 Kohlenstoffatomen in dem Alkylrest bedeuten,
R₁ für eine Bindung zu L oder ein Wasserstoffatom steht,
sowie Chelatbildnerreste der Formel II M,

Cp(aa)Cp- (II M)

worin
Cp ein geschütztes Cystein und (aa) für eine der natürlich vorkommenden Aminosäuren steht,
sowie cysteinreiche Aminosäuresequenzen der Metallothionine -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- und analoge Sequenzen, in denen Serin durch Threonin, Glycin oder Alanin ersetzt ist.

Die Reste E der erfindungsgemäßen Verbindungen der allgemeinen Formel (I)

E-L-(K)_{b} (I)

leiten sich bevorzugt von Endothelinen ab.

Von den Endothelinen ist das Endothelin 1 besonders bevorzugt, welches die folgende Aminosäuresequenz oder Teile davon aufweist:

Cys-Ser-Cys-Ser-Ser-Leu-Met-Asp-Lys-Glu-Cys-Val-Tyr-Phe-Cys-His-Leu-Asp-Ile-
Ile-Trp.

Die hydrophobe Region des Endothelin 1, His¹⁶-Trp²¹ ist für die Bindung an den Rezeptor essentiell (Kimura et al., 1988, Biochem. Biophys. Res. Comm., 156, 1182-1186).

Weiterhin bevorzugt sind Reste E, welche sich von Endothelin-Teilsequenzen ableiten.

Besonders bevorzugte Teilsequenzen der erfindungsgemäß eingesetzten Endotheline sind daher solche Peptide, welche die Aminosäuresequenz
-His-Leu-Asp-Ile-Ile-Trp- aufweisen.

Ferner sind Reste E bevorzugt, die sich von Endothelin-Analoga ableiten.

Besonders bevorzugte erfindungsgemäße Endothelin-Analoga weisen beispielsweise die folgenden Aminosäuresequenzen oder Teile davon auf:

-Cys-Ser-Cys-Ser-Ser-Trp-Leu-Asp-Lys-Glu-Cys-Val-Tyr-Phe-Cys-His-Leu-Asp-Ile-Ile-
Trp- (Endothelin 2).

-Cys-Thr-Cys-Phe-Thr-Tyr-Lys-Asp-Lys-Glu-Cys-Val-Tyr-Tyr-Cys-His-Leu-Asp-Ile-Ile-
Trp- (Endothelin 3).

-Cys-Ser-Ala-Ser-Ser-Leu-Met-Asp-Lys-Glu-Ala-Val-Tyr-Phe-Cys-His-Leu-Asp-Ile-Ile-
Trp- (Ala-Endothelin).

-Cys-Ser-Cys-Asn-Ser-Trp-Leu-Asp-Lys-Glu-Cys-Val-Tyr-Phe-Cys-His-Leu-Asp-Ile-Ile-
Trp- (VIC).

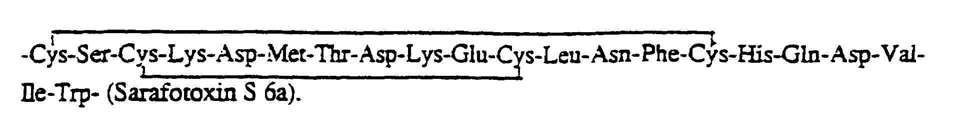
-Cys-Ser-Cys-Lys-Asp-Met-Thr-Asp-Lys-Glu-Cys-Leu-Asn-Phe-Cys-His-Gln-Asp-Val-
Ile-Trp- (Sarafotoxin S 6a).

-Ala-Ser-Cys-Ser-Ser-Leu-Met-Asp-Lys-Glu-Cys-Val-Tyr-Phe-Ala-His-Leu-Asp-Ile-Ile-
Trp-.

-Ala-Ser-Ala-Ser-Ser-Leu-Met-Asp-Lys-Glu-Ala-Val-Tyr-Phe-Ala-His-Leu-Asp-Ile-Ile-
Trp-.

-Cys-Ser-Cys-Ser-Ser-Trp-Leu-Asp-Lys-Glu-Ala-Val-Tyr-Phe-Ala-His-Leu-Asp-Ile-Ile-
Trp-.

Desweiteren bevorzugt sind Reste E, welche sich von Endothelin-Antagonisten ableiten. Bevorzugt eingesetzte Endothelin-Antagonisten sind zyklische Pentapeptide wie beispielsweise: -D Trp-D Asp-Pro-D Val-Leu- oder

-D Glu-Ala-allo D Ile-Leu-D Trp-.

Die erfindungsgemäßen Verbindungen der allgemeinen Formel (I)

E-L-(K)_{b} (I)

enthalten bevorzugt Reste L, welche geradkettig, verzweigt, zyklisch, aliphatisch, aromatisch oder arylaliphatisch sind und bis zu 20 Kohlenstoffatome aufweisen.

Bevorzugte Reste L stehen ferner für Z₁-R-Z₂,
worin
Z₁ und Z₂ unabhängig voneinander eine Gruppe -(C=O)O-, -(C=O)-, -NH-, -NH-(C=O)- bedeuten und
R eine geradkettige Mono- bis Dekamethylengruppe darstellt
oder einen Rest der Formel α 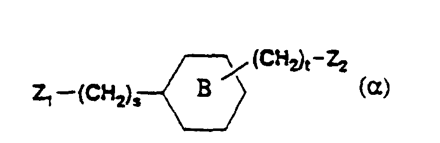 worin
s gleich 1 ist und t gleich 0 bedeutet und für den Ring B Phenylen und
Z₁ und Z₂ unabhängig voneinander für eine Gruppe -NH-(C=S)-, -NH-(C=S)NH-, -(C=O)O-, -(C=O)-, -NH-, -NH-(C=O)- bedeuten.

Bevorzugte Komplexbildnerreste K der erfindungsgemäßen Verbindungen der allgemeinen Formel (I)

E-L-(K)_{b} (I)

leiten sich von
S-Benzoylthioacetylglycylglycylglycin, N,N'-Bis(benzoylthioacetyl)-2,3-diaminopropionsäure, N,N'-Bis(benzoylthioacetyl)-3,4-diaminobuttersäure, N,N'-Bis(benzoylthioacetyl)-4,5-diaminopentansäure,
Cys(Acm)GlyCys(Acm)GlyGlyArgGlyAspSer, Ethylendiamintetraessigsäure, Diethylentriaminpentaessigsäure, trans-1,2-Cyclohexandiamintetraessigsäure, 1,4,7,10-Tetraazacyclododecantetraessigsäure, 1,4,7-Triazacyclononan-triessigsäure, 1,4,8,11-Tetraazatetradecantetraessigsäure, 1,5,9-Triazacyclododecantriessigsäure, 1,4,7,10-Tetraazacyclododecantriessigsäure und 3,6,9,15-Tetraazabicyclo[9,3,15]-pentadeca-1(15),11,13-trien-triessigsäure ab. Gegebenenfalls kann ein Teil der Carbonsäuren als Ester und/oder Amid vorliegen.

Sollen die Carbonsäuregruppen zumindest teilweise als Amide vorliegen, so sind tertiäre Amide bevorzugt. Als Reste kommen gesättigte, ungesättigte, gerad- oder verzweigtkettige oder zyklische Kohlenwasserstoffe mit bis zu 5 C-Atomen in Frage, die gegebenenfalls durch 1 bis 3 Hydroxy- oder C₁-C₄-Alkoxy-Gruppen substituiert sein können. Beispielsweise genannt seien die Methyl-, Ethyl-, 2-Hydroxyethyl-, 2-Hydroxy-1-(hydroxymethyl)-ethyl-, 1-(Hydroxymethyl)-ethyl, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethyl-Gruppe. Der Amidrest kann auch ein unter Einschluß des Amid-Stickstoffs gebildeter heterozyklischer 5- oder 6-Ring sein. Beispielhaft genannt seien: der Pyrrolidinyl-, Piperidinyl-, Pyrazolidinyl-, Pyrazolinyl-, Piperazinyl-, Morpholinyl-, Imidazolidinyl-, Oxazolidinyl-, Thiazolidinyl-Ring.

Die Herstellung der erfindungsgemäßen Komplexe von Verbindungen der allgemeinen Formel (I) mit Metallionen erfolgt nach den dem Fachmann bekannten Verfahren, in dem in an sich bekannter Weise ein radioaktives Metallion in Form seines Permetallates, in Anwesenheit eines Reduktionsmittels und gegebenenfalls eines Hilfsliganden mit einer Verbindung der allgemeinen Formel (I)

E-L-(K)_{b} (I)

umgesetzt wird.

Bevorzugte Metallionen sind ^{99m}Tc oder Re in Form von Pertechnat oder Perrhenat.

Die Reaktion wird bevorzugt in wäßrigem Medium bei Raumtemperatur durchgeführt. Die Abspaltung der SH-Schutzgruppen erfolgt in situ oder nach den dem Fachmann bekannten Literaturmethoden, zum Beispiel durch basische Hydrolyse, reduktive Spaltung, etc. (siehe zum Beispiel "Protective Groups in Organic Synthesis", T. W. Greene, John Wiley and Sons 1981).

Die Herstellung der erfindungsgemäßen Komplexe von Verbindungen der allgemeinen Formel (I) mit Metallionen erfolgt ferner auch dadurch, daß man in an sich bekannter Weise ein geeignetes Salz oder Oxid eines geeigneten paramagnetischen oder radioaktiven Kations mit einer Verbindung der allgemeinen Formel (I)

E-L-(K)_{b} (I)

umsetzt.

Bevorzugte radioaktive Metallionen sind beispielsweise ¹¹¹In, bevorzugte Metallionen mit paramagnetischen Eigenschaften sind die Ionen des Gd.

Der Einbau von paramagnetischen Kationen in die Chelatbildnerreste II K und II L erfolgt in literaturbekannter Weise (siehe z.B. DE 34 01 052 und EP 430863), in dem das Metalloxid oder Salz (z.B. das Nitrat, Acetat, Carbonat, Chlorid oder Sulfat) des jeweils gewünschten Metalls in polaren Lösungsmitteln wie Wasser oder wäßrigen Alkoholen suspendiert oder gelöst und mit der entsprechenden Menge des komplexbildenden Liganden umgesetzt wird. Soweit gewünscht, können vorhandene acide Wasserstoffatome oder Säuregruppen durch Kationen anorganischer und/oder organischer Basen oder Aminosäuren substituiert werden.

Geeignete anorganische Kationen sind beispielsweise das Lithiumion, das Kaliumion, das Kalziumion und insbesondere das Natriumion. Geeignete Kationen organischer Basen sind unter anderem solche von primären, sekundären oder tertiaren Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der ertindungsgemäßen Verbindungen der allgemeinen Formel (I) erfolgt in an sich bekannter Weise durch Umsetzung einer funktionellen Gruppe in E' welcher für ein Endothelin, ein Endothelinderivat, eine Endothelin-Teilsequenz, ein Endothelin-Analogon oder einen Endothelin-Antagonisten steht, mit einer funktionellen Gruppe in der allgemeinen Formel (III)

(K)_{b}-L-H (III)

wobei
L, K und b die oben genannte Bedeutung haben.

Die Herstellung der Verbindungen der Formel (I) erfolgt nach den dem Fachmann bekannten Verfahren durch Reaktion einer funktionellen Gruppe in L, beispielsweise mit einer terminalen oder ggf. einer Aminogruppe, einer entsprechend substituierten Seitenkette in E'. Die Verknüpfung von K' erfolgt kovalent an E' vorzugsweise über eine Amid- oder Thioamidbindung, mit E' in der Bedeutung von Endothelinen, Endothelinderivaten, Teilsequenzen von Endothelinen, Endothelin-Analoga oder Endothelin-Antagonisten, und mit K' in der Bedeutung der Chelatbildner der allgemeinen Formeln II A, II B und II K bis II M.

Erfolgt die Kopplung der Chelatbildner K' über eine Carboxylgruppe in L, so erfolgt die Umwandlung der Carboxylgruppe nach den dem Fachmann bekannten Verfahren zum Beispiei nach der Carbodiimid-Methode (Fieser, Reagents for Organic Synthesis 10, 142), über ein gemischtes oder zyklisches Anhydrid (Org. Prep. Proc. Int. 1975, 7, 215) oder über einen aktivierten Ester (Adv. Org. Chem. Part B, 472) und anschließender Reaktion mit einer nukleophilen Gruppe in E', vorzugsweise einer Aminogruppe, unter Ausbildung einer kovalenten Bindung.

Ebenfalls nach literaturbekannten Methoden werden isothiocyanat- und α-halogenacetylderivatisierte Komplexbildner bzw. Komplexe unter pH-Kontrolle in wäßrigem Milieu mit den gewünschten aminhaltigen E' in der Bedeutung von Endothelinen, Endothelinderivaten. Teilsequenzen von Endothelinen, Endothelin-Analoga oder Endothelin-Antagonisten umgesetzt.

Die Herstellung von E' erfolgt nach den von Borrany und Merrifield in The Peptides: Analysis, Biology (Pierce Chemical Co., Rochford, 31, 1984) beschriebenen Methoden.

Die Herstellung der Chelatbildner K' der allgemeinen Formel II A erfolgt in literaturbekannter Weise (vgl. EP 0248506) durch Chloracetylierung N-terminaler Aminogruppen von Di-, Tri-, Tetra-, Penta- oder Hexapeptiden und anschließender Umsetzung der entstandenen N-Chloracetylpeptide mit Alkalisalzen von Thiocarbonsäuren. Ein weiteres Verfahren zur Herstellung der Chelatbildner K' der allgemeinen Formel II A erfolgt in literaturbekannter Weise (vgl. EP 0248506) durch Reaktion entsprechender aktivierter (zum Beispiel NHS-Ester) und S-acylierter Thioessigsäurederivate oder 3-Thiopropionsäurederivate mit Di-, Tri-, Tetra-, Penta- oder Hexapeptiden. Die Aktivierung der entsprechenden Carbonsäuren erfolgt nach den dem Fachmann bekannten Verfahren zum Beispiel nach der Carbodiimid-Methode (Fieser, Reagents for Organic Synthesis 10, 142), über ein gemischtes oder zyklisches Anhydrid (Org. Prep. Proc. Int. 1975,7,215) oder über einen aktivierten Ester (Adv. Org. Chem. Part B, 472).

Die Herstellung der Chelatbildner K' der allgemeinen Formel II B erfolgt in literaturbekannter Weise (vgl. EP 0248506) durch Chloracetylierung der freien Aminogruppen von 1,2-Diaminopropionsäure oder 1,3-Diaminobuttersäure und anschließender Umsetzung der entstandenen N,N'-Dichloracetyl-diaminocarbonsäuren mit Alkalisalzen von Thiocarbonsäuren. Ein weiteres Verfahren zur Herstellung der Chelatbildner K' der allgemeinen Formel II B erfolgt in literaturbekannter Weise (siehe EP 0248506) durch Reaktion entsprechender aktivierter (zum Beispiel NHS-Ester) und S-acylierter Thioessigsäurederivate oder 3-Thiopropionsäurederivate mit 1,2-Diaminopropionsäure oder 1,3-Diaminobuttersäure.

Die Aktivierung der entsprechenden Carbonsäuren erfolgt nach den dem Fachmann bekannten Verfahren zum Beispiel nach der Carbodiimid-Methode (Fieser, Reagents for Organic Synthesis 10, 142), über ein gemischtes oder zyklisches Anhydrid (Org. Prep. Proc. Int. 1975, 7, 215) oder über einen aktivierten Ester (Adv. Org. Chem. Part B, 472).

Die Herstellung der Chelatbildner K' der allgemeinen Formel II K erfolgt in literaturbekannter Weise.

Die Herstellung der Chelatbildner K' der allgemeinen Formel II L erfolgt nach den dem Fachmann bekannten Verfahren, die beispielsweise in der Europäischen Patentanmeldung EP 0 512 661 beschrieben werden.

Die Herstellung der Chelatbildner K' der allgemeinen Formel II M erfolgt nach literaturbekannten Methoden der Festphasenpeptidsynthese (Barany and Merrifield, The Peptides: Analysis, Synthesis, Biology. Academic Press, New York, 1980; Stewart and Young, Solid-Phase Peptide Synthesis, 2nd ed., Pierce Chemical Co., Rockford, IL, 1984). Cysteine werden mit gewünschter S-Schutzgruppe eingesetzt. Die Peptide der Formel II M werden in N-geschützter Form vom Harz getrennt. Die Aktivierung des freien carboxyterminalen Endes erfolgt nach den dem Fachmann bekannten Verfahren zum Beispiel nach der Carbodiimid-Methode (Fieser, Reagents for Organic Synthesis 10, 142), über ein gemischtes oder zyklisches Anhydrid (Org. Prep. Proc. Int. 1975, 7, 215) oder über einen aktivierten Ester (Adv. Org. Chem. Part B, 472).

Die Herstellung cysteinreicher Aminosäuresequenzen von Metallothioninen (siehe Patentanmeldung WO 91/17173) erfolgt nach literaturbekannten Methoden der Festphasenpeptidsynthese (Barany and Merrifield, The Peptides: Analysis, Synthesis, Biology. Academic Press, New York, 1980; Stewart and Young, Solid-Phase Peptide Synthesis, 2nd ed., Pierce Chemical Co., Rockford, IL, 1984). Cysteine werden in S-geschützter Form eingesetzt. Die cysteinreichen Aminosäuresequenzen werden in N-geschützter Form vom Harz getrennt. Die Aktivierung des freien carboxyterminalen Endes erfolgt nach den dem Fachmann bekannten Verfahren zum Beispiel nach der Carbodiimid-Methode (Fieser, Reagents for Organic Synthesis 10, 142), über ein gemischtes oder zyklisches Anhydrid (Org. Prep. Proc. Int. 1975, 7, 215) oder über einen aktivierten Ester (Adv. Org. Chem. Part B, 472). Die Abspaltung der Schutzgruppen erfolgt nach der Kopplung an E' nach den dem Fachmann bekannten Literaturmethoden, zum Beispiel durch basische Hydrolyse, reduktive Spaltung, etc. (siehe zum Beispiel "Protective Groups in Organic Synthesis", T.W. Greene, John Wiley and Sons 1981).

Die vorliegende Erfindung stellt ferner diagnostische Mittel zur Verfügung, welche durch den Gehalt eines Komplexes der Verbindung der allgemeinen Formel (I) mit Metallionen der Ordnungszahlen 21-32, 37-39, 42-51 und 57-83 gekennzeichnet sind. Durch die geeignete Wahl des Metallions sind die Mittel für unterschiedliche diagnostische Verfahren geeignet.

Ist das erfindungsgemäße Mittel zur Anwendung in der Radiodiagnostik bestimmt, so muß das Zentralion des Komplexsalzes radioaktiv sein. Dies sind insbesondere die Ionen der Elemente der Ordnungszahlen 27, 29, 30-32, 37-39, 42-51, 62, 64, 70, 75 und 77. Bevorzugte Isotope sind beispielsweise ^{99m}Tc, ¹⁸⁶Re und ¹¹¹In.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29,42,44 und 57-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion, und die oben genannten Ionen der Lanthanidenreihe.

Ist das erfindungsgemäße Mittel zur Anwendung in der Positronen-Emissions-Tomographie bestimmt, so muß das Zentralatom ein positronenemittierendes Isotop sein. Dies sind insbesondere Isotope, wie z.B. ⁴³Sc, ⁴⁴Sc, ⁵²Fe, ⁵⁵Co, ⁶⁸Ga und Cu (Heiss, W. d.; Phelps M. E.; Positron Emission Tomography of Brain, Springer Verlag Berlin, Heidelberg, New York 1983).

Gegenstand der vorliegenden Erfindung sind ferner Verfahren zur Herstellung der erfindungsgemäßen diagnostischen Mittel.

Die Herstellung der erfindungsgemäßen radiopharmazeutischen Mittel erfolgt nach an sich bekannter Weise, in dem man die erfindungsgemäßen Komplexbildner und deren Konjugate - gegebenenfalls unter Zugabe der in der Galenik üblichen Zusätze - in wäßrigem Medium löst oder suspendiert und anschließend die Lösung oder Suspension gegebenenfalls lyophilisiert oder sterilisiert. Geeignete Zusätze sind beispielsweise physiologisch unbedenkliche Puffer (wie zum Beispiel Tromethamin), Zusätze von Hilfsliganden (wie zum Beispiel Natriumcitrat oder Natriumtartrat), Reduktionsmittel (wie zum Beispiel Zinn(II)-chlorid) oder - falls erforderlich - Elektrolyte wie zum Beispiel Natriumchlorid oder - falls erforderlich - (einen) in der Galenik üblichen Hilfsstoff(e) (zum Beispiel Lactose, Methylcellulose, Mannit) und/oder Tensid(en) (zum Beispiel Lecithine, Tween®, Myrj®). Die verwendeten Zusätze müssen in ihrer Zusammensetzung eine Herstellung der erfindungsgemäßen Verbindungen erlauben.

Bei der nuklearmedizinischen *in vivo*-Anwendung werden die erfindungsgemäßen Mittel in Mengen von 1x10⁻⁵ bis 5x10⁴ nmol/kg Körpergewicht, vorzugsweise in Mengen zwischen 1x10⁻³ bis 5x10² nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Die Applikation erfolgt normalerweise durch intravenöse, intraarterielle oder peritoneale Injektion von 0,1 bis 5 ml einer Lösung der erfindungsgemäßen Mittel. Bevorzugt ist die intravenöse Applikation. Details ihrer Anwendung und Dosierung werden zum Beispiel in "Radiotracers for Medical Applications", CRC-Press, Boca Raton, Florida, beschrieben. Die erfindungsgemäßen Verbindungen kommen zur Anwendung für die Radiodiagnostik und Radiotherapie in Form ihrer Komplexe mit den Radioisotopen der Elemente mit der Ordnungszahl 27, 29, 30-32, 37-39, 42-51, 62, 64, 70, 75 und 77.

Die erfindungsgemäßen radiopharmazeutischen Mittel erfüllen die vielfältigen Voraussetzungen für die Eignung als Radiopharmaka für die Radiodiagnostik und die Radiotherapie. So sind sie hervorragend dazu geeignet, sich nach i.v. Applikation in Zielgeweben anzureichern und ermöglichen so eine nicht-invasive Diagnose entsprechender Gewebe. Die Wasserlöslichkeit der erfindungsgemäßen radiopharmazeutischen Mittel wird - falls erforderlich - durch die in der Galenik üblichen Hilfsstoffe wie oben beschrieben gewährleistet.

Weiterhin weisen die erfindungsgemäßen radiopharmazeutischen Mittel nicht nur eine hohe Stabilität *in vitro* auf, sondern auch eine überraschend hohe Stabilität *in vivo,* so daß eine Freigabe oder ein Austausch des im Komplex gebundenen Radionuklids nicht oder klinische nicht relevant erfolgt.

Die Herstellung der erfingungsgemäßen pharmazeutischen Mittel für die NMR- und Röntgen-Diagnostik erfolgt ebenfalls in an sich bekannter Weise, indem man die erfindungsgemäßen Komplexverbindungen -gegebenenfalls unter Zugabe der in der Galenik üblichen Zusätze- in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Die erfingungsgemäßen pharmazeutischen Mittel für die NMR-Diagnostik enthalten vorzugsweise 1µMol - 1 Mol/l des Komplexsalzes und werden in der Regel in Mengen von 0,001 - 5 mMol/kg dosiert. Sie sind zur enteralen und parenteralen Applikation bestimmt. Die erfindungsgemäßen Komplexverbindungen kommen zur Anwendung für die NMRund Röntgen-Diagnostik in Form ihrer Komplexe mit den Ionen der Elemente mit den Ordnungszahlen 21-29, 42, 44 und 57-83.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nicht-invasiven Charakter der Untersuchungen aufrechtzuerhalten.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Die erfindungsgemäßen Mittel sind hervorragend als Röntgenkontrastmittel geeignet, wobei besonders hervorzuheben ist, daß sich mit ihnen keine Anzeichen der von den iodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Subtraktionstechniken.

Die Herstellung der erfindungsgemäßen pharmazeutischen Mittel erfolgt ferner in an sich bekannter Weise, indem man die erfindungsgemäßen Komplexbildner unter Zusatz eines Reduktionsmittels, vorzugsweise Zinn-(II)-salzen wie -chlorid oder -tartrat und - gegebenfalls unter Zugabe der in der Galenik üblichen Zusätze - in wäßrigem Medium löst und anschließend sterilfiltriert. Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Zur Herstellung der Radiophannaka wird ein erfindungsgemäßes Cold Kit zur Verfügung gestellt. Dieses Cold Kit beinhaltet eine erfindungsgemäße Verbindung der allgemeinen Formel (I), ein Reduktionsmittel und gegebenenfalls einen oder mehrere Hilfsliganden in Lösung, in trockenem Zustand oder in lyophilisierter Form. Das Cold Kit umfaßt ferner eine Gebrauchsanweisung mit einer Reaktionsvorschrift zur Umsetzung der erfindungsgemäßen Verbindungen der allgemeinen Formel (I) mit einem Permetallat eines radioaktiven Metallions unter Bildung eines erfindungsgemäßen Komplexes einer Verbindung der allgemeinen Formel (I) mit dem Metallion.

Die Verwendung dieses Cold Kits, das eine ausreichende Menge eines Reduktionsmittels enthält, um die Verbindung mit ^{99m}Tc zu markieren zur Herstellung einer radiopharmazeutischen Zubereitung ist ebenfalls ein Gegenstand der vorliegenden Erfindung.

Gegenstand der vorliegenden Erfindung ist ferner ein Verfahren zur bildlichen Darstellung pathologischer Gefäßveränderungen, dadurch gekennzeichnet, daß als Kontrastmittel ein Komplex der Verbindung der allgemeinen Formel (I) mit Metallionen der Ordnungszahlen 21-32, 37-39, 42-51 und 57-83, eingesetzt wird.

Das erfindungsgemäße Verfahren zur bildlichen Darstellung pathologischer Gefäßveränderungen ist ferner dadurch gekennzeichnet, daß als Kontrastmittel ein radioaktiv markiertes Endothelin eingesetzt wird.

In einer Methode zur Durchführung einer radiodiagnostischen Untersuchung wird die radiopharmazeutische Zusammensetzung in einer Menge von 0,1 bis 30 mCi, bevorzugt von 0,5 bis 10 mCi pro 70 kg Körpergewicht einem Patienten verabreicht und die vom Patienten abgegebene Strahlung aufgezeichnet.

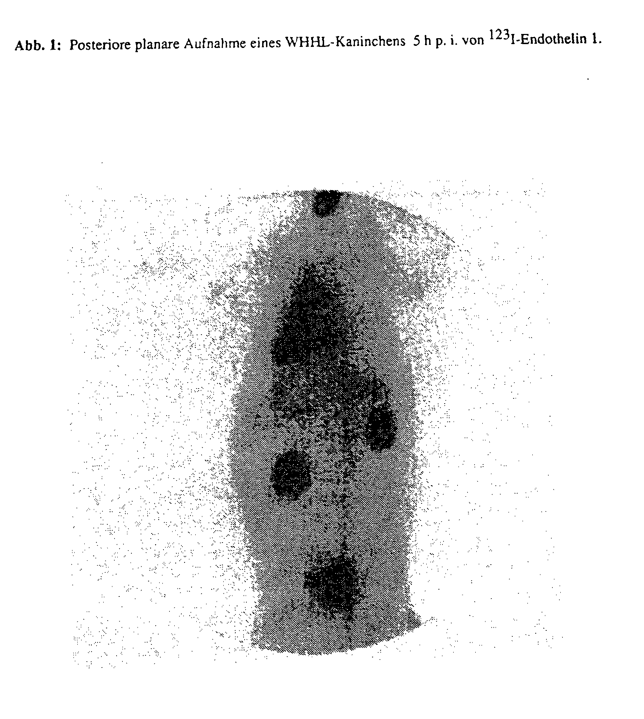

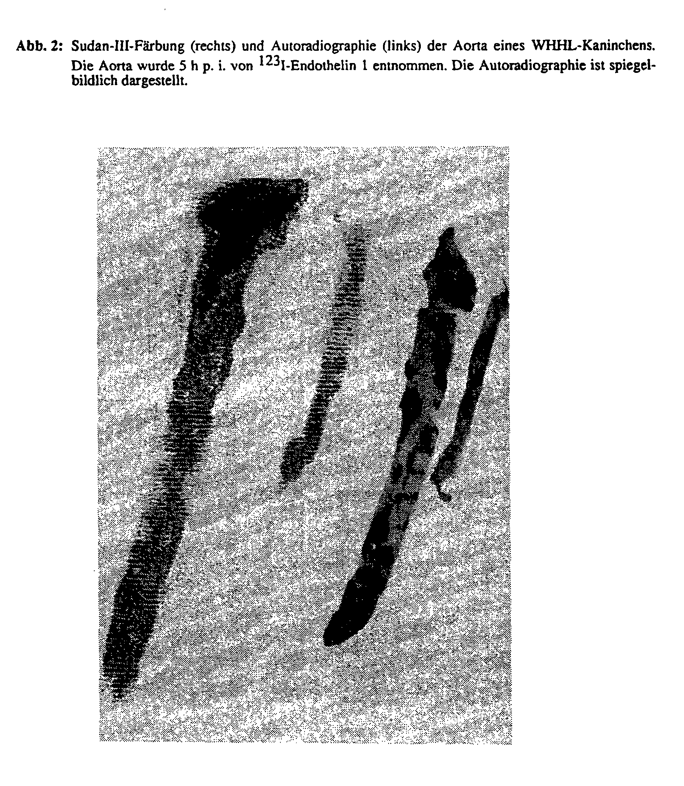

Das erfindungsgemäße Verfahren zur bildlichen Darstellung pathologischer Gefäßveränderungen wird durch die Abbildungen 1 und 2 erläutert.

Abb. 1 zeigt eine posteriore planare Aufnahme eines WHHL-Kaninchens 5 h p.i. von ¹²³I-Endothelin 1.

Abb. 2 zeigt eine Sudan-III-Färbung sowie eine Autoradiographie der Aorta eines WHHL-Kaninchens. Die Aorta wurde 5 h p.i. von ¹²³I-Endothelin 1 entnommen.

Die nachfolgenden Beispiele dienen der näheren Erläuterung des Erfindungsgegenstandes.

### Beispiel 1

a) S-Benzoylthioacetyl-Gly-Gly-Gly-Gly-Asp-His-Leu-Asp-Ile-Ile-Trp.
   Eine Lösung aus 50 mg S-Benzoylthioacetyl-Gly-Gly-Gly und 16 mg N-Hydroxysuccinimid in absolutem frisch destilliertem Dimethylformamid wird auf -15°C gekühlt und mit 29 mg Dicyclohexylcarbodiimid in absolutem Dimethylformamid versetzt Die Reaktionsmischung wird 2 Stunden bei -5°C, anschließend 2 Stunden bei Raumtemperatur gerührt und schließlich auf -15°C abgekühlt. Ausgefallener N,N'-Dicyclohexylharnstoff wird abfiltriert. Das Filtrat wird mit einer Lösung aus 1 mg Gly-Asp-His-Leu-Asp-Ile-Ile-Trp in absolutem Dimethylformamid versetzt und 20 Stunden bei Raumtemperatur gerührt. Die Reaktionslösung wird im Vakuum auf ein Minimum eingeengt. Nach Zutropfen von Diethylether resultiert ein flockiger Niederschlag, der durch Zentrifugation isoliert und anschließend über präparative HPLC (Gradient: Acetonitril/Phosphatpuffer) gereinigt wird. Nach der Neutralisation der gepufferten Lösung wird der organische Lösungsmittelanteil mit N₂ abgeblasen und der Rückstand gefriergetrocknet.
   - Molekulargewicht:: ber. 1301.4 best. 1301 (FAB-MS)
b) ^{99m}Tc-Komplex von S-Benzoylthioacetyl-Gly-Gly-Gly-Gly-Asp-His-Leu-Asp-Ile-Ile-Trp.
   Eine Lösung von 0.5 mg des S-Benzoylthioacetyl-Gly-Gly-Gly-Gly-Asp-His-Leu-Asp-Ile-Ile-Trp in 300 µl Phosphatpuffer (Na₂HPO₄, 0.1 mol/l, pH 9.5) wird mit 50 µl einer 0.15 molaren Trinatriumcitratdihydrat-Lösung und 2.5 µl einer 0.2 molaren Zinn(II)chlorid-Dihydrat-Lösung versetzt. Das Reaktionsgemisch wird mit einer Pertechnetatlösung (0.4-0.9 mCi) aus einem Mo-99/Tc-99m-Generator versetzt, 15 min bei Raumtemperatur inkubiert und anschließend filtriert (0.2 µ-Filter). Die Analytik der Markierung erfolgt über HPLC: MERCK Nucleosil-Säule, 125x4 mm, 5 um; Gradient von 100% A nach 100% B innerhalb von 7.5 min. Eluent A: Phosphatpuffer (Na₂HPO₄; 0.01 M; pH 2.0); Eluent B: Acetonitril /Phosphatpuffer (Na₂HPO₄; 0.01 M; pH 2.0) 75:25 (V/V); Flußrate: 1.0 ml/min.

### Beispiel 2

a) 
N,N´-Bis(S-benzoylthioacetyl)-3,4-diaminobutyryl-Cys-Ser-Cys-Ser-Ser-Leu-Met-Asp-
Lys-Glu-Cys-Val-Tyr-Phe-Cys-His-Leu-Asp-Ile-Ile-Trp.
   Eine Lösung aus 50 mg N,N'-Bis(S-benzoylthioacetyl)-3,4-diaminobuttersäure und 15 mg N-Hydroxysuccinimid in absolutem frisch destilliertem Dimethylformamid wird auf -15°C gekühlt und mit 28 mg Dicyclohexylcarbodiimid in absolutem Dimethylformamid versetzt. Die Reaktionsmischung wird 2 Stunden bei -5°C, anschließend 2 Stunden bei Raumtemperatur gerührt und schließlich auf -15°C abgekühlt. Ausgefallener N,N'-Dicyclohexylharnstoff wird abfiltriert. Das Filtrat wird mit einer Lösung aus 1 mg Cys-Ser-Cys-Ser-Ser-Leu-Met-Asp-Lys-Glu-Cys-Val-Tyr-Phe-Cys-His-Leu-Asp-Ile-Ile-Trp (Endothelin 1) in absolutem Dimethylformamid versetzt und 20 Stunden bei Raumtemperatur gerührt. Die Reaktionslösung wird im Vakuum auf ein Minimum eingeengt. Nach Zutropfen von Diethylether resultiert ein flockiger Niederschlag, der durch Zentrifugation isoliert und anschließend über präparative HPLC (Gradient: Acetonitril/Phosphatpuffer) gereinigt wird. Nach der Neutralisation der gepufferten Lösung wird der organische Lösungsmittelanteil mit N₂ abgeblasen und der Rückstand gefriergetrocknet.
   - Molekulargewicht:: ber. 2948.5
   best 2949 (FAB-MS)
b) 
99mTc-Komplex von N,N´-Bis(S-benzoylthioacetyl)-3,4-diaminobutyryl-Cys-Ser-Cys-
Ser-Ser-Leu-Met-Asp-Lys-Glu-Cys-Val-Tyr-Phe-Cys-His-Leu-Asp-Ile-Ile-Trp.
   Die Markierung von N,N'-Bis(S-benzoylthioacetyl)-3,4-diaminobutyryl-Cys-Ser-Cys-Ser-Ser-Leu-Met-Asp-Lys-Glu-Cys-Val-Tyr-Phe-Cys-His-Leu-Asp-Ile-Ile-Trp erfolgt analog Beispiel 2b.

### Beispiel 3

a) 
N,N´-Bis(S-benzoylthioacetyl)-4,5-diamino-1-oxo-pentyl-Cys-Ser-Cys-Ser-Ser-Leu-
Met-Asp-Lys-Glu-Cys-Val-Tyr-Phe-Cys-His-Leu-Asp-Ile-Ile-Trp.
   Eine Lösung aus 50 mg N,N'-Bis(S-benzoylthioacetyl)-4,5-diaminopentansäure und 15 mg N-Hydroxysuccinimid in absolutem frisch destilliertem Dimethylformamid wird auf -15°C gekühlt und mit 28 mg Dicyclohexylcarbodiimid in absolutem Dimethylformamid versetzt. Die Reaktionsmischung wird 2 Stunden bei -5°C, anschließend 2 Stunden bei Raumtemperatur gerührt und schließlich auf -15°C abgekühlt. Ausgefallener N,N'-Dicyclohexylharnstoff wird abfiltriert. Das Filtrat wird mit einer Lösung aus 1 mg Cys-Ser-Cys-Ser-Ser-Leu-Met-Asp-Lys-Glu-Cys-Val-Tyr-Phe-Cys-His-Leu-Asp-Ile-Ile-Trp (Endothelin 1) in absolutem Dimethylformamid versetzt und 20 Stunden bei Raumtemperatur gerührt. Die Reaktionslösung wird im Vakuum auf ein Minimum eingeengt. Nach Zutropfen von Diethylether resultiert ein flockiger Niederschlag, der durch Zentrifugation isoliert und anschließend über präparative HPLC (Gradient: Acetonitril/Phosphatpuffer) gereinigt wird. Nach der Neutralisation der gepufferten Lösung wird der organische Lösungsmittelanteil mit N₂ abgeblasen und der Rückstand gefriergetrocknet.
   - Molekulargewicht:: ber. 2962.5
   best. 2963 (FAB-MS)
b) ^{99m}Tc-Komplex von N,N'-Bis(S-benzoylthioacetyl)-4,5-diamino-1-oxo-pentyl-Cys-Ser-Cys-Ser-Ser-Leu-Met-Asp-Lys-Glu-Cys-Val-Tyr-Phe-Cys-His-Leu-Asp-Ile-Ile-Trp.
   Die Markierung von N,N'-Bis(S-benzoylthioacetyl)-4,5-diamino-1-oxo-pentyl-Cys-Ser-Cys-Ser-Ser-Leu-Met-Asp-Lys-Glu-Cys-Val-Tyr-Phe-Cys-His-Leu-Asp-Ile-Ile-Trp erfolgt analog Beispiel 2b.

### Beispiel 4

a) N-[3,6,9-Triaza-1-oxo-3,6,9-tris(hydroxycarbonylmethyl)-9-(ethoxycarbonylinethyl)-nonyl]-Gly-His-Leu-Asp-Ile-Ile-Trp.
Zu einer Lösung von 853 mg (1 mmol) NH₂-Gly-His-Leu-Asp-Ile-Ile-Trp (hergestellt in Analogie zu Barany und Marrifield, The Peptides: Analysis, Biology, Academic Press, New York, 1980; Stewart und Young, Solid-Phase Peptide Synthesis, 2nd ed., Pierce Chemical Co., Rockford, IL, 1984) und 506 mg (5 mmol) Triethylamin in 100 ml absolutem Dimethylformamid gibt man portionsweise 403,4 mg (1 mmol) 3-Ethoxycarbonylmethyl-6-[2-(2,6-dioxomorpholino)-ethyl]-3,6-diaza-octandisäure (DTPA-monoethylester-monoanhydrid). Man rührt 24 h bei Raumtemperatur. Nach beendeter Reaktion wird filtriert und das Lösungsmittel im Vakuum abgezogen. Das verbleibende Öl wird dreimal mit 50 ml Dimethylformamid versetzt und jeweils eingedampft. Man verrührt den Rückstand mit 200 ml absolutem Diethylether, worauf sich ein weißer Feststoff abscheidet, der abfiltriert wird. Dieser wird aus Dimethylformamid/Diethylethergemischen umkristallisiert.
Ausbeute: 634 mg (50,4 %), weißes Pulver.

| Analyse: bezogen auf die wasserfreie Substanz | | | | |
|---|---|---|---|---|
| Ber. | C 54,49 | H 6,82 | N 14,49 | O 24,20 |
| Gef. | C 54,23 | H 7,03 | N 14,34 | |

b) Synthese von N-[3,6,9-Triaza-1-oxo-3,6,9,9-tetra-(hydroxycarbonylmethyl)-nonyl]-Gly-His-Leu-Asp-Ile-Ile-Trp.
1,26 g (1 mmol) des hergestellten N-[3,6,9-Triaza-1-oxo-3,6,9-tris-(hydroxycarbonylmethyl)-9-(ethoxycarbonylmethyl)-nonyl]-Gly-His-Leu-Asp-Ile-Ile-Trp (Beispiel 10a) werden in 100 ml Wasser suspendiert und durch Zugabe von 10 molarer, wässriger Natriumhydroxidlösung wird ein pH-Wert von 12,5 eingestellt. Man rührt 5 h bei Raumtemperatur, stellt durch Zugabe von konzentrierter Salzsäure einen pH-Wert von 2 ein, filtriert das rohe Konjugat ab und wäscht mit wenig Eiswasser und eisgekühltem Methanol nach. Zur Reinigung wird in wenig Wasser bei pH 7 aufgenommen und an Kieselgel RP-18 (Eluens: Wasser/Tetrahydrofuran, Tetrahydrofuran: 0-50 %) chromatographiert. Die das Konjugat enthaltenden Fraktionen werden eingedampft und der Rückstand in wenig Wasser bei pH 4,5 aufgenommen. Durch Zugabe von konzentrierter Salzsäure wird das Produkt bei pH 2 gefällt und unter vermindertem Druck getrocknet.
Ausbeute: 213 mg (17,3 %), weißes Pulver.

| Analyse: bezogen auf die wasserfreie Substanz | | | | |
|---|---|---|---|---|
| Ber. | C 53,78 | H 6,65 | N 14,82 | O 24,75 |
| Gef. | C 53,49 | H 6,91 | N 14,57 | |

Eine Variante zur Herstellung des N-[3,6,9-Triaza-1-oxo-3,6,9,9-tetra-(hydroxycarbonylmethyl)-nonyl]-Gly-His-Leu-Asp-Ile-Ile-Trp (Beispiel 10b) besteht darin, daß 853 mg (1 mmol) NH₂-Gly-His-Leu-Asp-Ile-Ile-Trp (Beispiel 10a), gelöst in 100 ml Wasser, bei pH 9 portionsweise mit 3,57 g (10 mmol) Bis-(aminoethyl)-N, N, N', N", N"' -pentaessigsäuredianhydrid umgesetzt wird. Man rührt 3 h bei Raumtemperatur; dabei ist durch tropfen-weise Zugabe von 1 N wässriger Natriumhydroxidlösung der pH-Wert des Reaktionsme-diums stets bei 9 zu halten. Anschließend stellt man durch Zugabe von konzentrierter Salzsäure einen pH-Wert von 2 ein, filtriert das rohe Konjugat ab und wäscht mit wenig Eiswasser und eisgekühltem Methanol nach. Zur Reinigung wird in wenig Wasser bei pH 7 aufgenommen und an Kieselgel RP-18 (Eluens: Wasser/Tetrahydrofuran, Tetrahydrofuran: 0-50 %) chromatographiert. Die das Konjugat enthaltenden Fraktionen werden eingedampft und der Rückstand in wenig Wasser bei pH 4,5 aufgenommen. Durch Zugabe von konzentrierter Salzsäure wird das Produkt bei pH 2 gefällt und unter vermindertem Druck getrocknet.
Ausbeute: 317 mg (25,7 %), weißes Pulver.
c) ^{99m}Tc-Komplex von N-[3,6,9-Triaza-1-oxo-3,6,9,9-tetra-(hydroxycarbonylmethyl)-nonyl]-Gly-His-Leu-Asp-Ile-Ile-Trp.
Eine Lösung von 0,5 mg des hergestellten N-[3,6,9-Triaza-1-oxo-3,6,9,9-tetra-(hydroxycarbonylmethyl)-nonyl]-Gly-His-Leu-Asp-Ile-Ile-Trp (Beispiel 10b) in 300 µl Phosphatpuffer (Na₂HPO₄, 0,5 mol/l, pH 7,0) wird mit 50 µl einer 0,15 molaren Trinatriumcitratdihydrat-Lösung und 2,5 µl einer 0,2 molaren Zinn(II)-Chlorid-Dihydrat-Lösung versetzt. Das Reaktionsgemisch wird mit einer Pertechnetatlösung (0,4 - 0,9 mCi) aus einem Mo-99/Tc-99m-Generator versetzt, 10 min bei Raumtemperatur inkubiert und anschließend filtriert (0,2 µ-Filter). Die Analytik der Markierung erfolgt über HPLC: MERCK Nucleosil-Säule, 125 x 4 mm, 5 µm; Gradient von 100 % A nach 100 % B innerhalb von 7,5 min. Eluent A: Phosphatpuffer (Na₂HPO₄); 0,01 M; pH 2,0) 50:50 (V/V); Flußrate: 1,0 ml/min.
d) ¹¹¹In-Komplex von N-[3,6,9-Triaza-1-oxo-3,6,9,9-tetra-(hydroxycarbonylmethyl)-nonyl]-Gly-His-Leu-Asp-Ile-Ile-Trp.
Eine Lösung von 0,5 mg des hergestellten N-[3,6,9-Triaza-1-oxo-3,6,9,9-tetra-(hydroxycarbonylmethyl)-nonyl]-Gly-His-Leu-Asp-Ile-Ile-Trp (Beispiel 10b) in 300 µl Phosphatpuffer (Na₂HPO₄, 0,5 mol/l, pH 6,5) wird mit 20 µl ¹¹¹InCl₃ (3 MBq, NEN, Du Pont) für 10 min bei Raumtemperatur inkubiert und anschließend filtriert (0,2 µ-Filter). Die Analytik der Markierung erfolgt über HPLC : MERCK Nucleosil-Säule, 125 x 4 mm, 5 µm; Gradient von 100 % A nach 100 % B innerhalb von 7,5 min. Eluent A: Phosphatpuffer (Na₂HPO₄); 0,01 M; pH 2,0) 50 : 50 (V/V); Flußrate: 1,0 ml/min.
e) Gd(III)-Komplex von N-[3,6,9-Triaza-1-oxo-3,6,9,9-tetra-(hydroxycarbonylmethyl)-nonyl]-Gly-His-Leu-Asp-Ile-Ile-Trp, Natriumsalz.
Zu einer Lösung von 2,46 g (2 mmol) des hergestellten N-[3,6,9-Triaza-1-oxo-3,6,9,9-tetra-(hydroxycarbonylmethyl)-nonyl]-Gly-His-Leu-Asp-Ile-Ile-Trp (Beispiel 10b) in 100 ml Wasser bei pH 6,5 gibt man portionsweise 669 mg (2 mmol) Gadolinium-(III)-acetat. Dabei wird durch Zugabe von 10 N wässriger Natriumhydroxidlösung der pH-Wert des Reaktionsgemisches stets zwischen 6 und 6,5 gehalten. Ist alles Gadolinium-(III)-acetat zugesetzt, wird noch 4 h bei Raumtemperatur nachgerührt. Nach beendeter Komplexierung stellt man durch Zutropfen von 1 N wäßriger Salzsäurelösung einen pH-Wert von 7 ein und filtriert. Die klare Lösung wird gefriergetrocknet. Der Rückstand wird in wenig Wasser aufgenommen und an Kieselgel RP-18 (Eluens: Wasser/Tetrahydrofuran; Trahydrofüran: 0-50 %) chromatographiert. Die das Produkt enthaltenden Fraktionen werden am Rotationsverdampfer von Tetrahydrofuran befreit und die Restlösung gefriergetrocknet.
Ausbeute: 1,13 mg (39,6%), weißes Pulver.

| Analyse: bezogen auf die wasserfreie Substanz | | | | | | |
|---|---|---|---|---|---|---|
| Ber. | C 46,31 | H 5,37 | N12,76 | O 21,31 | Gd 11,02 | Na 3,22 |
| Gef. | C 46,09 | H 5,63 | N 12,49 | | Gd 10,83 | Na 3,51 |

### Beispiel. 5

### Gd(III)-Komplex von 1-{2-Hydroxy-3-[4-(GIy-His-Leu-Asp-Ile-Ile-Trp-thiouridyl)phenoxy]propyl}1,4,7,10-tetraaza-4,7,10-tris-(carboxylatomethyl)-cyclododecan.

Zu einer Lösung von 853 mg (1 mmol) NH₂-Gly-His-Leu-Asp-Ile-Ile-Trp (hergestellt in Analogie zu Barany und Marrifield, The Peptides: Analysis, Biology, Academic Press, New York, 1980; Stewart und Young, Solid-Phase Peptide Synthesis, 2nd ed., Pierce Chemical Co., Rockford, IL, 1984) und 304 mg (3 mmol) Triethylamin in 100 ml absolutem Dimethylformamid unter Argonatmosphäre gibt man portionsweise 708 mg (1 mmol) 1-[2-Hydroxy-3-(4-isothiocyanato-phenoxy)-propyl]-1,4,7,10-tetraaza-4,7,10-tris-(carboxylatomethyl)-cyclododecan, Gd-Komplex (hergestellt nach EP 0 485 045). Man rührt 24 h bei Raumtemperatur. Nach beendeter Reaktion wird filtriert und das Lösungsmittel im Vakuum abgezogen. Das verbleibende Öl wird dreimal mit 50 ml Dimethylformamid versetzt und jeweils eingedampft. Man verrührt den Rückstand mit 200 ml absolutem Diethylether, worauf sich ein weißer Feststoff abscheidet, der abfiltriert wird. Dieser wird aus Dimethylformamid/Diethylethergemischen umkristallisiert.

Ausbeute: 533 mg (34,0 %), weißes Pulver.

| Analyse: bezogen auf die wasserfreie Substanz | | | | | | |
|---|---|---|---|---|---|---|
| Ber. | C 50,02 | H 5,94 | N 13,46 | O 18,45 | S 2,05 | Gd 10,07 |
| Gef. | C 49,78 | H 6,23 | N 13,17 | | S 1,89 | Gd 9,83 |

### Beispiel 6

a) 
Cys(Acm)-Gly-Cys(Acm)-Cys-Ser-Cys-Ser-Ser-Leu-Met-Asp-Lys-Glu-Cys-Val-Tyr-
Phe-Cys-His-Leu-Asp-Ile-Ile-Trp.
   Eine Lösung aus 50 mg Fmoc-Cys(Acm)-Gly-Cys(Acm) und 15 mg N-Hydroxysuccinimid in absolutem frisch destilliertem Dimethylformamid wird auf -15°C gekühlt und mit 28 mg Dicyclohexylcarbodiimid in absolutem Dimethylformamid versetzt. Die Reaktionsmischung wird 2 Stunden bei -5°C, anschließend 2 Stunden bei Raumtemperatur gerührt und schließlich auf -15°C abgekühlt. Ausgefallener N,N'-Dicyclohexylharnstoff wird abfiltriert. Das Filtrat wird mit einer Lösung aus 1 mg Cys-Ser-Cys-Ser-Ser-Leu-Met-Asp-Lys-Glu-Cys-Val-Tyr-Phe-Cys-His-Leu-Asp-Ile-Ile-Trp (Endothelin 1) in absolutem Dimethylformamid versetzt und 20 Stunden bei Raumtemperatur gerührt. Zur Abspaltung der Schutzgruppe wird die Reaktionslösung wird mit etwas Piperidin versetzt, 30 min bei Raumtemperatur gerührt und anschließend im Vakuum auf ein Minimum eingeengt Nach Zutropfen von Diethylether resultiert ein flockiger Niederschlag, der durch Zentrifugation isoliert und anschließend über präparative HPLC (Gradient: Acetonitril/Phosphatpuffer) gereinigt wird. Nach der Neutralisation der gepufferten Lösung wird der organische Lösungsmittelanteil mit N₂ abgeblasen und der Rückstand gefriergetrocknet.
   - Molekulargewicht:: ber. 2897.4
   best. 2898 (FAB-MS)
b) 
⁹⁹ᵐTc-Komplex von Cys(Acm)-Gly-Cys(Acm)-Cys-Ser-Cys-Ser-Ser-Leu-Met-Asp-
Lys-Glu-Cys-Val-Tyr-Phe-Cys-His-Leu-Asp-Ile-Ile-Trp.
   Die Markierung von Cys(Acm)-Gly-Cys(Acm)-Cys-Ser-Cys-Ser-Ser-Leu-Met-Asp-Lys-Glu-Cys-Val-Tyr-Phe-Cys-His-Leu-Asp-Ile-Ile-Trp erfolgt analog Beispiel 2 b.

### Beispiel 7

a) Cyclo(Trp-Leu-Val-Pro-Asp)-Cys(Acm)-Gly-Cys(Acm).
   Eine Lösung von 133.0 mg Cyclo(Trp-Leu-Val-Pro-Asp) (Herstellung in Analogie zu EP 0,436,189) und 41.3 mg Hydroxybenzotriazol in 6 ml absolutem frisch destilliertem Dimethylformamid wird auf -15°C gekühlt und mit einer Lösung von 51.7 mg 1-Ethyl-3-(3-dimethylaminopropyl)carbodiimidhydrochlorid in 8 ml Dimethylformamid versetzt. Die Reaktionsmischung wird 2 Stunden bei -5°C gerührt. Man rührt weitere 2h bei Raumtemperatur und tropft langsam eine Lösung von 134.0 mg Cys(Acm)-Gly-Cys(Acm)-O-t-But in absolutem Dimethylformamid zu. Nach weiteren 7h Rühren wird im Vakuum eingeengt Vorhandene Schutzgruppen werden mit Triflouressigsäure abgespalten, das Peptid mit Ether gefällt und mittels präparativer HPLC (Gradient: Acetonitril/Phosphatpuffer) gereinigt. Nach der Neutralisation der gepufferten Lösung wird der organische Lösungsmittelanteil mit N₂ abgeblasen und der Rückstand gefriergetrocknet.
   - Molekulargewicht:: ber. 1016.2
   best. 1016 (FAB-MS)
b) ^{99m}Tc-Komplex von Cyclo(Trp-Leu-Val-Pro-Asp)-Cys(Acm)-Gly-Cys(Acm).
   Die Markierung von Cyclo(Trp-Leu-Val-Pro-Asp)-Cys(Acm)-Gly-Cys(Acm) erfolgt analog Beispiel 2b.

### Beispiel 8

a) 
4-Mercaptobutyrimidyl-Cys-Ser-Ala-Ser-Ser-Leu-Met-Asp-Lys-Glu-Ala-Val-Tyr-Phe-
Cys-His-Leu-Asp-Ile-Ile-Trp.
   Eine Lösung von 500µg Cys-Ser-Ala-Ser-Ser-Leu-Met-Asp-Lys-Glu-Ala-Val-Tyr-Phe-Cys-His-Leu-Asp-Ile-Ile-Trp (Ala-Endothelin) in 400µl Phosphat gepufferte Saline (pH 7,4) wird mit dem 100 fachem molaren Überschuß an Trauts-Reagenz (2,6 mg Iminothiolan in 400µl Triethanolaminhydrochlorid-Puffer pH 8,0) eine Stunde bei Raumtemperatur inkubiert. Anschließend wird die Reaktion beendet, indem auf einer Gelfiltrationssäule (SEC 3000 Beckman) überschüssiges Trauts-Reagenz abgetrennt wird. Eine Kontrolle der Umsetzung erfolgt über die Bestimmung der freien SH-Gruppen (nach Grasseti).
   - Molekulargewicht:: ber. 2504.9
   best. 2505 (FAB-MS)
b) 
⁹⁹ᵐTc-Komplex von 4-Mercaptobutyrimidyl-Cys-Ser-Ala-Ser-Ser-Leu-Met-Asp-Lys-
Glu-Ala-Val-Tyr-Phe-Cys-His-Leu-Asp-Ile-Ile-Trp.
   Eine Lösung von 0.5 mg 4-Mercaptobutyrimidyl-Cys-Ser-Ala-Ser-Ser-Leu-Met-Asp-Lys-Glu-Ala-Val-Tyr-Phe-Cys-His-Leu-Asp-Ile-Ile-Trp in 300µl Phosphatpuffer (Na₂HPO₄, 0.5 mol/l, pH 9.5) wird mit 50 µl einer 0.15 molaren Trinatriumcitratdihydrat-Lösung und 2.5 µl einer 0.2 molaren Zinn(II)chlorid-Dihydrat-Lösung versetzt. Das Reaktionsgemisch wird mit einer Pertechnetatlösung (0.4-0.9 mCi) aus einem Mo-99/Tc-99m-Generator versetzt, 10 min bei Raumtemperatur inkubiert und anschließend filtriert (0.2 µ-Filter). Die Analytik der Markierung erfolgt über HPLC: MERCK Nucleosil-Säule, 125x4 mm, µm; Gradient von 100% A nach 100% B innerhalb von 7.5 min. Eluent A: Phosphatpuffer (Na₂HPO₄; 0.01 M; pH 2.0); Eluent B:Acetonitril/Phosphatpuffer (Na₂HPO₄; 0.01 M; pH 2.0) 75:25 (V/V); Flußrate: 1.0 ml/min.

### Beispiel 9

a) 3-Thiopropionyl-Gly-Asp-His-Leu-Asp-Ile-Ile-Trp.
   Eine Lösung von 500µg Gly-Asp-His-Leu-Asp-Ile-Ile-Trp in 400µl Phosphat gepufferter Saline (pH 8,5) wird mit dem 10 fachem molaren Überschuß an N-Succinimidyl-3-(2-pyridyldithio)propionat in Dimethylformamid versetzt und zwei Stunden bei Raumtemperatur inkubiert Anschließend wird die vorhandene 2-Pyridyldisulfidgruppe mit 25 mM Dithiothreitol, pH 4.5 reduziert. Die Reaktionslösung wird im Vakuum auf ein Minimum eingeengt. Anschließend wird über präparative HPLC (Gradient: Acetonitril/Phosphatpuffer) gereinigt. Nach der Neutralisation der gepufferten Lösung wird der organische Lösungsmittelanteil mit N₂ abgeblasen und der Rückstand gefriergetrocknet.
   - Molekulargewicht:: ber. 1040.2
   best. 1040 (FAB-MS)
b) ^{99m}Tc-Komplex von 3-Thiopropionyl-Gly-Asp-His-Leu-Asp-Ile-Ile-Trp.
   Die Markierung von 3-Thiopropionyl-Gly-Asp-His-Leu-Asp-Ile-Ile-Trp erfolgt analog Beispiel 14 b.

### Beispiel 10

a) 2-(Acetylthio)succinyl-Gly-Asp-His-Leu-Asp-Ile-Ile-Trp.
   Eine Lösung von 500 µg Gly-Asp-His-Leu-Asp-Ile-Ile-Trp in 50 µl Dimethylformamid wird mit dem 10 fachem molaren Überschuß an 2-Mercaptoacetylbernsteinsäureanhydrid in 5 µl Dimethylformamid versetzt und zwei Stunden bei Raumtemperatur inkubiert. Anschließend wird über präparative HPLC (Gradient: Acetonitril/Wasser) gereinigt. Der organische Lösungsmittelanteil wird mit N₂ abgeblasen und der Rückstand gefriergetrocknet.
   - Molekulargewicht:: ber. 1126.2
   best. 1126 (FAB-MS)
b) ^{99m}Tc-Komplex von 2-(Acetylthio)succinyl-Gly-Asp-His-Leu-Asp-Ile-Ile-Trp.
   Die Markierung von 2-(Acetylthio)succinyl-Gly-Asp-His-Leu-Asp-Ile-Ile-Trp erfolgt analog Beispiel 14 b.

## Patentansprüche

1. Komplexe von Verbindungen der allgemeinen Formel (I)
E-L-(K)_{b} (I)
mit Metallionen der Ordnungszahlen 21-32, 37-39, 42-51 und 57-83,
worin
E für einen Rest, abgeleitet von Endothelinen, Endothelinderivaten, Endothelin-Teilsequenzen, Endothelin-Analoga oder Endothelin-Antagonisten steht,
und wobei der Rest E die Eigenschaft aufweist, selektiv an Endothelin-Rezeptoren zu binden,
L eine direkte Bindung oder einen Rest Z₁-R-Z₂ darstellt,
worin
R für einen gegebenenfalls durch ein oder mehrere Sauerstoff- und/oder Schwefelatome und/oder Carbonyl-, -NHCO-, -N(C₁₋₆Alkyl)CO-, -NHund -N(C₁₋₆Alkyl)-Reste unterbrochenen und gegebenenfalls durch Hydroxy-und/oder Epoxygruppen substituierten geradkettigen, verzweigten, gesättigten oder ungesättigten C₁₋₂₀ -Alkylrest steht,
Z₁ und Z₂ unabhängig voneinander eine Gruppe -O-, -S-, -(C=O)O-, -NH-(C=S)NH-, -(C=O)-, -(C=S)O-, -NH-, -NH-(C=O)- oder -NH-(C=S)- ist,
oder für einen Rest der Formel α 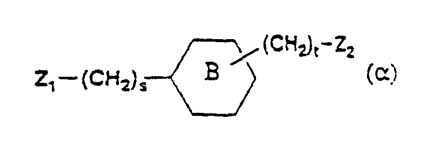 steht, worin
s und t voneinander unabhängig für die Ziffern 0, 1, 2 oder 3 stehen, der Ring B einen Phenyl- oder Cyclohexylrest bedeutet und Z₁ und Z₂ die vorstehend angegebene Bedeutung haben,
b für die Ziffer 1 steht,
K einen Chelatbildnerrest der allgemeinen Formel II A oder II B 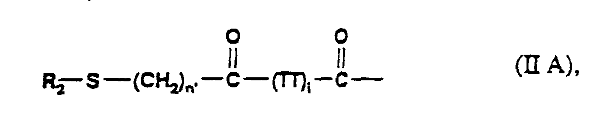 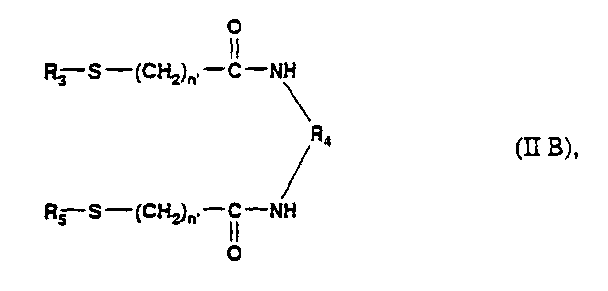
darstellt,
worin
R₂, R₃ und R₅ unabhängig voneinander für ein Wasserstoffatom, einen gegebenenfalls mit einer Hydroxylgruppe, einem C₁₋₄-Alkoxy-, einem Carboxyl- oder einem Sulfonsäurerest substituierten (C₁₋₆-Alkyl)CO-, (C₆₋₈-Aryl)CO- oder (C₇₋₉-Arylalkyl)CO-Rest stehen und
R₄ für einen Rest der Formel II C oder II D 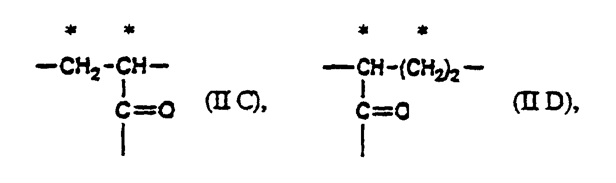 worin
die mit einem * gekennzeichneten Kohlenstoffatome an die Iminogruppen der Formel II B gebunden sind und
n' für die Ziffern 1 oder 2,
i für eine beliebige Zahl von 2 bis 6, und
TT für α- und/oder β- Aminosäuren, welche in üblicher Weise über Amidgruppen verbunden sind,
stehen,
sowie Chelatbildnerreste, die sich von Aminopolycarbonsäuren der Formel II K oder II L 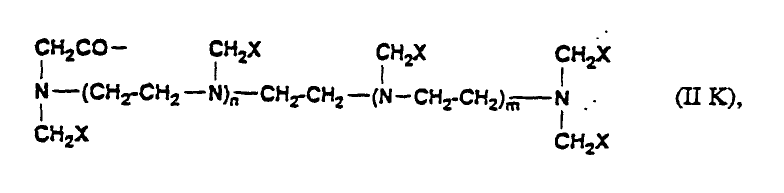 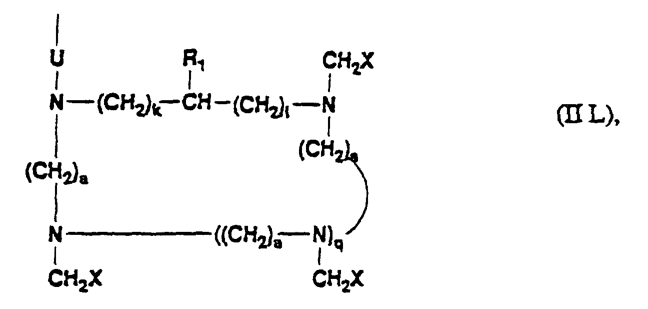 ableiten, wobei
n und m jeweils für die Ziffern 0, 1, 2, 3 oder 4 stehen, wobei n und m nicht mehr als 4 ergeben,
a für die Ziffern 2, 3, 4, oder 5
k für die Ziffern 1, 2, 3, 4, oder 5,
l für die Ziffern 0, 1, 2, 3, 4, oder 5 und
q für die Ziffern 0, 1 oder 2 stehen,
U ein Wasserstoffatom oder einen C₁₋₆-Alkylrest darstellt, welcher gegebenenfalls mit einer oder mehreren Hydroxy-Gruppen substituiert ist und eine Bindung zu L enthält,
die Reste X unabhängig voneinander jeweils Wasserstoffatome, COOH-Gmppen, Estergruppen oder Amidgruppen mit 1-6 Kohlenstoffatomen in dem Alkylrest bedeuten,
R₁ für eine Bindung zu L oder ein Wasserstoffatom steht,
sowie Chelatbildnerreste der Formel II M,
Cp(aa)Cp- (II M)
worin
Cp für ein geschütztes Cystein und (aa) für eine der natürlich vorkommenden Aminosäuren steht,
sowie cysteinreiche Aminosäuresequenzen der Metallothionine -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- und analoge Sequenzen, in denen Serin durch Threonin, Glycin oder Alanin ersetzt ist.

2. Verbindungen nach Anspruch 1, **dadurch gekennzeichnet, daß** das Metallion M ein Ion eines der Isotope von Tc, Re, In, Cr, Mn, Fe, Co, Ni, Cu, Pr, Nd, Sm,Y, Gd, Tb. Dy, Ho, Er und La ist.

3. Verbindungen nach mindestens einem der Ansprüche 1 bis 2, **dadurch gekennzeichnet, daß** die Endotheline E die Aminosäuresequenz Cys-Ser-Cys-Ser-Ser-Leu-Met-Asp-Lys-Glu-Cys-Val-Tyr-Phe-Cys-His-Leu-Asp-Ile-Ile-Trp oder Teile davon aufweisen.

4. Verbindungen nach mindestens einem der Ansprüche 1 bis 2, **dadurch gekennzeichnet, daß** die Teile der Endotheline E die Aminosäuresequenz -His-Leu-Asp-Ile-Ile-Trp- aufweisen.

5. Verbindungen nach mindestens einem der Ansprüche 1 bis 2, **dadurch gekennzeichnet, daß** die Endothelin-Analoga E eine der Aminosäuresequenzen 
-Cys-Ser-Cys-Ser-Ser-Trp-Leu-Asp-Lys-Glu-Cys-Val-Tyr-Phe-Cys-His-Leu-Asp-Ile-
Ile-Trp-;
 
-Cys-Thr-Cys-Phe-Thr-Tyr-Lys-Asp-Lys-Glu-Cys-Val-Tyr-Tyr-Cys-His-Leu-Asp-
Ile-Ile-Trp-;
 
-Cys-Ser-Ala-Ser-Ser-Leu-Met-Asp-Lys-Glu-Ala-Val-Tyr-Phe-Cys-His-Leu-Asp-Ile-
Ile-Trp-;
 
-Cys-Ser-Cys-Asn-Ser-Trp-Leu-Asp-Lys-Glu-Cys-Val-Tyr-Phe-Cys-His-Leu-Asp-
Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-Asp-Ile-
Ile-Trp-;
 
-Ala-Ser-Ala-Ser-Ser-Leu-Met-Asp-Lys-Glu-Ala-Val-Tyr-Phe-Ala-His-Leu-Asp-Ile-
Ile-Trp-;
 
-Cys-Ser-Cys-Ser-Ser-Trp-Leu-Asp-Lys-Glu-Ala-Val-Tyr-Phe-Ala-His-Leu-Asp-Ile-
Ile-Trp-;
 oder Teile davon aufweisen.

6. Verbindungen nach mindestens einem der Ansprüche 1 bis 2, **dadurch gekennzeichnet, daß** die Endothelin -Antagonisten E eines der zyklischen Pentapeptide -D Trp-D Asp-Pro-D Val-Leu- oder -D Glu-Ala-allo D Ile-Leu-D Trp- sind.

7. Verbindungen nach mindestens einem der Ansprüche 1 bis 6, **dadurch gekennzeichnet daß** die für L stehende Alkylengruppe geradkettig, verzweigt, zyklisch, aliphatisch, aromatisch oder arylaliphatisch ist.

8. Verbindungen nach mindestens einem der Ansprüche 1 bis 6, **dadurch gekennzeichnet, daß** L für Z₁-R-Z₂ steht,
worin
Z₁ und Z₂ unabhängig voneinander eine Gruppe -(C=O)O-, -(C=O)-, -NH-, -NH-(C=O)- bedeuten und
R eine geradkettige Mono- bis Dekamethylengruppe darstellt
oder einen Rest der Formel α 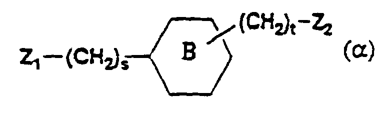 bedeutet, worin
s gleich 1 ist und t gleich 0 bedeutet und für den Ring B Phenylen und
Z₁ und Z₂ unabhängig voneinande für eine Gruppe -NH-(C=S)-, -NH-(C=S)NH-, -(C=O)O-, -(C=O)-, -NH-, -NH-(C=O)- stehen.

9. Verbindungen nach mindestens einem der Ansprüche 1 bis 8, **dadurch gekennzeichnet, daß** der Komplexbildnerrest K
einen S-Benzoylthioacetylglycylglycylglycinrest,
einen N,N'-Bis(benzoylthioacetyl)-2,3-diaminopropionsäurerest,
einen N,N'-Bis(benzoylthioacetyl)-3,4-diaminobuttersäurerest,
einen N,N'-Bis(benzoylthioacetyl)-4,5-diaminopentansäurerest,
einen Cys(Acm)-Gly-Cys(Acm)-Gly-Gly-Arg-Gly-Asp-Ser-Rest,
einen Ethylendiamintetraessigsäurerest,
einen Diethylentriaminpentaessigsäurerest,
einen trans-1, 2-Cyclohexandiamintetraessigsäurerest,
einen 1, 4, 7, 10-Tetraazacylclododecantetraessigsäurerest,
einen 1, 4, 7-Triazacyclononan-triessigsäurerest,
einen 1, 4, 8, 11-Tetraazatetradecantetraessigsäurerest,
einen 1, 5, 9-Triazacylclododecantriessigsäurerest,
einen 1, 4, 7, 10-Tetraazacyclododecantriessigsäurerest oder
einen 3, 6, 9, 15-Tetraazabicyclo[9, 3, 15]-pentadecal (15), 11, 13-trien-triessigsäurerest darstellt.

10. Komplexe von Verbindungen der allgemeinen Formel (I) mit Metallionen gemäß Definition in Anspruch 1, nämlich
a) ^{99m}Tc-Komplex von S-Benzoylthioacetyl-Gly-Gly-Gly-Gly-Asp-His-Leu-Asp-Ile-Ile-Trp,
b) 
⁹⁹ᵐTc-Komplex von N,N´-Bis(S-benzoylthioacetyl)-3,4-diaminobutyryl-Cys-Ser-
Cys-Ser-Ser-Leu-Met-Asp-Lys-Glu-Cys-Val-Tyr-Phe-Cys-His-Leu-Asp-Ile-Ile-
Trp,
c) 
⁹⁹ᵐTc-Komplex von N,N´-Bis(S-benzoylthioacetyl)-4,5-diamino-1-oxo-pentyl-
Cys-Ser-Cys-Ser-Ser-Leu-Met-Asp-Lys-Glu-Cys-Val-Tyr-Phe-Cys-His-Leu-Asp-
Ile-Ile-Trp,
d) ^{99m}Tc-Komplex von N-[3,6,9-Triaza-1-oxo-3,6,9,9-tetra-(hydroxycarbonylmethyl)-nonyl]-Gly-His-Leu-Asp-Ile-Ile-Trp,
e) ¹¹¹In-Komplex von N-[3,6,9-Triaza-1-oxo-3,6,9,9-tetra-(hydroxycarbonylmethyl)-nonyl]-Gly-His-Leu-Asp-Ile-Ile-Trp,
f) Gd(III)-Komplex von N-[3,6,9-Triaza-1-oxo-3,6,9,9-tetra-(hydroxycarbonylmethyl)-nonyl]-Gly-His-Leu-Asp-Ile-Ile-Trp, Natriumsalz,
g) Gd(III)-Komplex von 1-{2-Hydroxy-3-[4-(Gly-His-Leu-Asp-Ile-Ile-Trp-thiouridyl)phenoxy]propyl} 1,4,7,10-tetraaza-4,7,10-tris-(carboxylatomethyl)-cyclododecan,
h) 
99mTc-Komplex von Cys(Acm)-Gly-Cys(Acm)-Cys-Ser-Cys-Ser-Ser-Leu-Met-
Asp-Lys-Glu-Cys-Val-Tyr-Phe-Cys-His-Leu-Asp-Ile-Ile-Trp,
i) ^{99m}Tc-Komplex von Cyclo(Trp-Leu-Val-Pro-Asp)-Cys(Acm)-Gly-Cys(Acm).
k) 
99mTc-Komplex von 4-Mercaptobutyrimidyl-Cys-Ser-Ala-Ser-Ser-Leu-Met-Asp-
Lys-Glu-Ala-Val-Tyr-Phe-Cys-His-Leu-Asp-Ile-Ile-Trp,
l) ^{99m}Tc-Komplex von 3-Thiopropionyl-Gly-Asp-His-Leu-Asp-Ile-Ile-Trp,
m) ^{99m}Tc-Komplex von 2-(Acetylthio)succinyl-Gly-Asp-His-Leu-Asp-Ile-Ile-Trp.

11. Verbindungen der allgemeinen Formel (I)
E-L-(K)_{b} (I)
worin
E für einen Rest, abgeleitet von Endothelinen, Endothelinderivaten, Endothelin-Teilsequenzen, Endothelin-Analoga oder Endothelin-Antagonisten, steht,
und wobei der Rest E die Eigenschaft aufweist, selektiv an Endothelin-Rezeptoren zu binden,
L eine direkte Bindung oder einen Rest Z₁-R-Z₂ darstellt,
worin
R für einen gegebenenfalls durch ein oder mehrere Sauerstoff- und/oder Schwefelatome und/oder Carbonyl, -NHCO-, -N(C₁₋₆Alkyl)CO-, -NHund -N(C₁₋₆-Alkyl)-Reste unterbrochenen und gegebenenfalls durch Hydroxy-und/oder Epoxygruppen substimierten geradkettigen, verzweigten, gesätrigten oder ungesätrigten C₁₋₂₀ Alkylrest steht,
Z₁ und Z₂ unabhängig voneinander eine Gruppe -O-, -S-, -(C=O)O-, -NH-(C=S)NH-, -(C=O)-, -(C=S)O-, -NH-, -NH-(C=O)- oder -NH-(C=S)-ist,
oder für einen Rest der Formel α 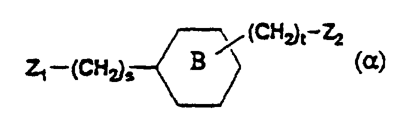 steht, worin
s und t voneinander unabhängig für die Ziffern 0, 1, 2 oder stehen, der Ring B einen Phenyl- oder Cyclohexylrest bedeutet und Z₁ und Z₂ die vorstehend angegebene Bedeutung haben,
b für die Ziffer 1 steht,
K einen Chelatbildnerrest der allgemeinen Formel II A oder II B 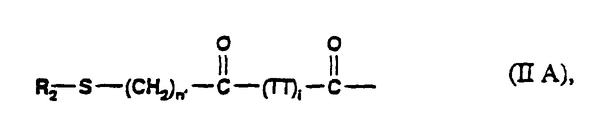 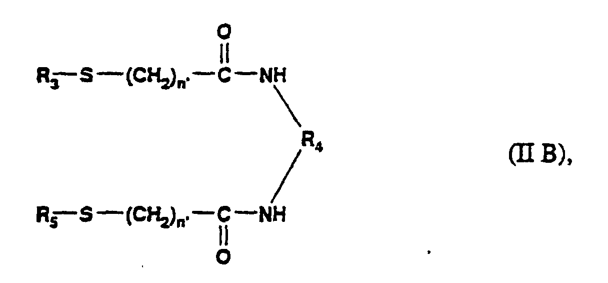
darstellt,
worin
R₂, R₃ und R₅ unabhängig voneinander für ein Wasserstoffatom, einen gegebenenfalls mit einer Hydroxylgruppe, einem C₁₋₄-Alkoxy-, einem Carboxyl- oder einem Sulfonsäurerest substituierten (C₁₋₆-Alkyl)CO-, (C₆₋₈-Aryl)CO- oder (C₇₋₉-Arylalkyl)CO-Rest stehen und
R₄ für einen Rest der Formel II C oder II D 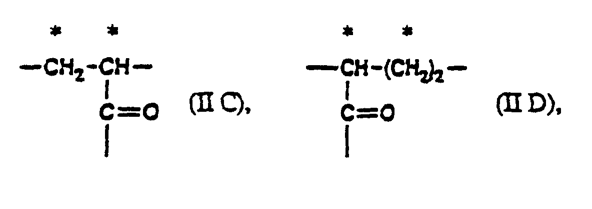 worin
die mit einem * gekennzeichneten Kohlenstoffatome an die Iminogruppen der Formel II B gebunden sind und
n' für die Ziffern 1 oder 2,
i für eine beliebige Zahl von 2 bis 6, und
TT für α- und/oder β- Aminosäuren, welche in üblicher Weise über Amidgruppen verbunden sind,
stehen,
sowie Chelatbildnerreste, die sich von Aminopolycarbonsäuren der Formel II K oder II L 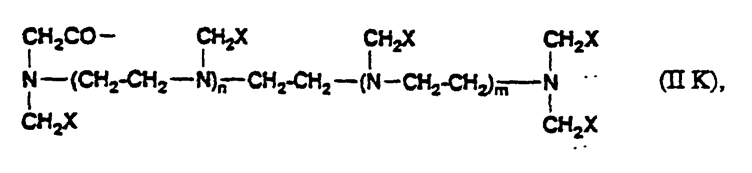 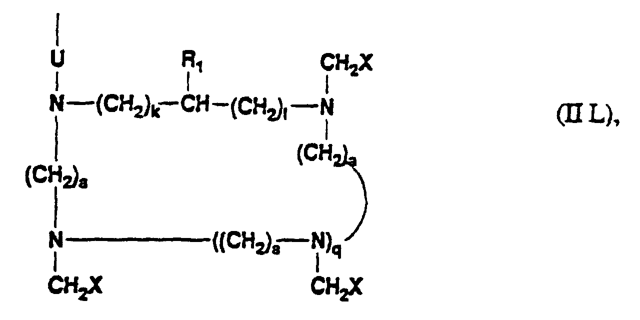 ableiten, wobei
n und m jeweils für die Ziffern 0, 1, 2, 3 oder 4 stehen, wobei n und m nicht mehr als 4 ergeben,
a für die Ziffern 2,3,4, oder 5
k für die Ziffern 1, 2, 3, 4, oder 5,
l für die Ziffern 0, 1, 2, 3, 4, oder 5 und
q für die Ziffern 0, 1 oder 2 stehen,
U ein Wasserstoffatom oder einen C₁₋₆-Alkylrest darstellt, welcher gegebenenfalls mit einer oder mehreren Hydroxy-Gruppen substituiert ist und eine Bindung zu L enthält,
die Reste X unabhängig voneinander jeweils Wasserstoffatome, COOH-Gruppen. Estergruppen oder Amidgruppen mit 1-6 Kohlenstoffatomen in dem Alkylrest bedeuten,
R₁ für eine Bindung zu L oder ein Wasserstoffatom steht,
sowie Chelatbildnerreste der Formel II M,
Cp(aa)Cp- (II M)
worin
Cp ein geschütztes Cystein und (aa) für eine der natürlich vorkommenden Aminosäuren steht,
sowie cysteinreiche Aminosäuresequenzen der Metallothionine -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- und analoge Sequenzen, in denen Serin durch Threonin, Glycin oder Alanin ersetzt ist.

12. Verbindungen nach Anspruch 11, **dadurch gekennzeichnet, daß** die Endotheline E die Aminosäuresequenz Cys-Ser-Cys-Ser-Ser-Leu-Met-Asp-Lys-Glu-Cys-Val-Tyr-Phe-Cys-His-Leu-Asp-Ile-Ile-Trp oder Teile davon aufweisen.

13. Verbindungen nach Anspruch 11, **dadurch gekennzeichnet, daß** die Teile der Endotheline E die Aminosäuresequenz -His-Leu-Asp-Ile-Ile-Trp- aufweisen.

14. Verbindungen nach Anspruch 11, **dadurch gekennzeichnet, daß** die Endothelin-Analoga E eine der Aminosäuresequenzen. 
-Cys-Ser-Cys-Ser-Ser-Trp-Leu-Asp-Lys-Glu-Cys-Val-Tyr-Phe-Cys-His-Leu-
Asp-Ile-Ile-Trp-;
 
-Cys-Thr-Cys-Phe-Thr-Tyr-Lys-Asp-Lys-Glu-Cys-Val-Tyr-Tyr-Cys-His-Leu-
Asp-Ile-Ile-Trp-;
 
-Cys-Ser-Ala-Ser-Ser-Leu-Met-Asp-Lys-Glu-Ala-Val-Tyr-Phe-Cys-His-Leu-
Asp-Ile-Ile-Trp-;
 
-Cys-Ser-Cys-Asn-Ser-Trp-Leu-Asp-Lys-Glu-Cys-Val-Tyr-Phe-Cys-His-Leu-
Asp-Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-
Asp-Ile-Ile-Trp-;
 
-Ala-Ser-Ala-Ser-Ser-Leu-Met-Asp-Lys-Glu-Ala-Val-Tyr-Phe-Ala-His-Leu-
Asp-Ile-Ile-Trp-;
 
-Cys-Ser-Cys-Ser-Ser-Trp-Leu-Asp-Lys-Glu-Ala-Val-Tyr-Phe-Ala-His-Leu-
Asp-Ile-Ile-Trp-;
 oder Teile davon aufweisen.

15. Verbindungen nach Anspruch 11, **dadurch gekennzeichnet, daß** die Endothelin-Antagonisten E eines der zyklischen Pentapeptide -D Trp-D Asp-Pro-D Val-Leu- -D Glu-Ala-allo D Ile-Leu-D Trp- sind.

16. Verbindungen nach mindestens einem der Ansprüche 11 bis 15 **dadurch gekennzeichnet, daß** die für L stehende Alkylengruppe geradkettig, verzweigt, zyklisch, aliphatisch, aromatisch oder arylaliphatisch ist.

17. Verbindungen nach mindestens einem der Ansprüche 11 bis 15 **dadurch gekennzeichnet, daß** L für Z₁-R-Z₂ steht,
worin
Z₁ und Z₂ unabhängig voneinander eine Gruppe -(C=O)O-, -(C=O)-, -NH-, -NH-(C=O)- bedeuten und
R eine geradkettige Mono- bis Dekamethylengruppe darstellt
oder einen Rest der Formel α 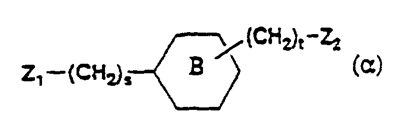 bedeutet, worin
s gleich 1 ist und t gleich 0 bedeutet und für den Ring B Phenylen und
Z₁ und Z₂ unabhängig voneinander für eine Gruppe -NH-(C=S)-, -NH-(C=S)NH-, -(C=O)O-, -(C=O)-, -NH-, -NH-(C=O)- stehen.

18. Verbindungen nach mindestens einem der Ansprüche 11 bis 17, **dadurch gekennzeichnet, daß** der Komplexbildnerrest K
einen S-Benzoylthioacetylglycylglycylglycinrest,
einen N,N'-Bis(benzoylthioacetyl)-2,3-diaminopropionsäurerest,
einen N,N'-Bis(benzoylthioacetyl)-3,4-diaminobuttersäurerest,
einen N,N'-Bis(benzoylthioacetyl)-4,5-diaminopentansäurerest,
einen Cys(Acm)-Gly-Cys(Acm)-Gly-Gly-Arg-Gly-Asp-Ser-Rest,
einen Ethylendiamintetraessigsäurerest,
einen Diethylentriaminpentaessigsäurerest,
einen trans-1, 2-Cyclohexandiamintetraessigsäurerest,
einen 1, 4, 7, 10-Tetraazacylclododecantetraessigsäurerest,
einen 1, 4, 7-Triazacyclononan-triessigsäurerest,
einen 1, 4, 8, 11-Tetraazatetradecantetraessigsäurerest,
einen 1, 5, 9-Triazacylclododecantriessigsäurerest,
einen 1, 4, 7, 10-Tetraazacyclododecantriessigsäurerest oder
einen 3, 6, 9, 15-Tetraazabicyclo[9,3,15]-pentadecal (15), 11, 13-trien-triessigsäurerest darstellt.

19. Verfahren zur Herstellung der Komplexe von Verbindungen der allgemeinen Formel (I) mit Metallionen, wie definiert in Anspruch 1
**dadurch gekennzeichnet,**
**daß** man in an sich bekannter Weise ein radioaktives Metallion in Form seines Permetallates, in Anwesenheit eines Reduktionsmittels und gegebenenfalls eines Hilfsliganden mit einer Verbindung der allgemeinen Formel (I) gemäß Anspruch 1
E-L-(K)_{b} (I)
umsetzt.

20. Verfahren zur Herstellung der Komplexe von Verbindungen der allgemeinen Formel (I) mit Metallionen, wie definiert in Anspruch 1
**dadurch gekennzeichnet,**
**daß** man in an sich bekannter Weise ein geeignetes Salz oder Oxid eines geeigneten paramagnetischen und/oder eines radioaktiven Kations mit einer Verbindung der allgemeinen Formel (I) gemäß Anspruch 1
E-L-(K)_{b} (I)
umsetzt.

21. Verfahren nach Anspruch 19,
**dadurch gekennzeichnet,**
**daß** man Technetium-99m oder Re in Form von Pertechnetat oder Perrhenat einsetzt.

22. Verfahren nach Anspruch 20,
**dadurch gekennzeichnet,**
**daß** man als radioaktives Kation ¹¹¹In einsetzt.

23. Verfahren nach Anspruch 20,
**dadurch gekennzeichnet,**
**daß** man als paramagnetisches Kation Gd einsetzt.

24. Verfahren zur Herstellung einer Verbindung der allgemeinen Formel (I),
**dadurch gekennzeichnet,**
**daß** man in an sich bekannter Weise E', welcher für ein Endothelin, ein Endothelinderivat, eine Endothelin-Teilsequenz, ein Endothelin-Analogon oder einen Endothelin-Antagonisten steht, mit einer Verbindung der Formel (III)
(K)_{b}-L-H (III)
umsetzt, wobei K, L und b die in Anspruch 1 angegebene Bedeutung haben.

25. Verfahren nach Anspruch 24,
**dadurch gekennzeichnet,**
**daß** die Verknüpfung zwischen E und L über eine Ester-, Ether-, Thioether-, Thioester-, Amid- oder Thioamidbindung vorliegt.

26. Cold Kit zur Herstellung von Radiopharmaka, bestehend aus einer Verbindung der allgemeinen Formel (1) nach einem der Ansprüche 11 bis 18, einem Reduktionsmittel und gegebenenfalls einem Hilfsliganden, die in trockenem Zustand oder in Lösung vorliegen, einer Gebrauchsanweisung mit einer Reaktionsvorschrift zur Umsetzung der beschriebenen Verbindung mit Technetium-99m oder Re in Form einer Pertechnetatlösung oder Perrhenatlösung.

27. Verwendung einer Verbindung gemäß einem der Ansprüche 1 bis 10 zur Herstellung von Mitteln für die Diagnose von Gefäßerkrankungen.

28. Verwendung einer Verbindung gemäß einem der Ansprüche 11 bis 18 zur Herstellung von Mitteln für die Diagnose von Gefäßerkrankungen unter Verwendung von Metallionen M gemäß Anspruch 2.

## Claims

1. Complexes of compounds of the general formula (I)
E-L-(K)_{b} (I)
with metal ions of atomic numbers 21-32, 37-39, 42-51 and 57-83,
in which
E is a radical derived from endothelins, endothelin derivatives, endothelin part-sequences, endothelin analogues or endothelin antagonists,
and where the radical E has the property of binding selectively to endothelin receptors,
L is a direct linkage or a Z₁-R-Z₂ radical,
in which
R is a straight-chain, branched, saturated or unsaturated C₁₋₂₀-alkyl radical which is optionally interrupted by one or more oxygen and/or sulphur atoms and/or carbonyl, -NHCO-, -N(C₁₋₆alkyl)CO-, -NH- and -N (C₁₋₆alkyl)-radicals and optionally substituted by hydroxyl and/or epoxy groups,
Z₁ and Z₂ are, independently of one another, a -O-, -S-, -(C=O)O-, -NH-(C=S)NH-, -(C=O)-, -(C=S)O-, -NH-, -NH-(C=O)- or -NH-(C=S)- group,
or a radical of the formula α 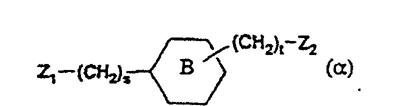 in which
s and t are independently of one another, the numbers 0, 1, 2 or 3, the ring B is a phenyl or cyclohexyl radical, and Z₁ and Z₂ have the meaning indicated above,
b is the number 1,
K is a chelating agent radical of the general formula II A or II B 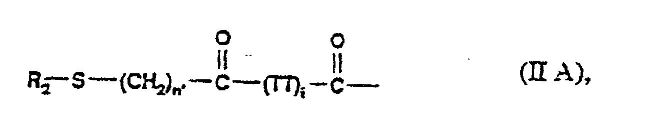 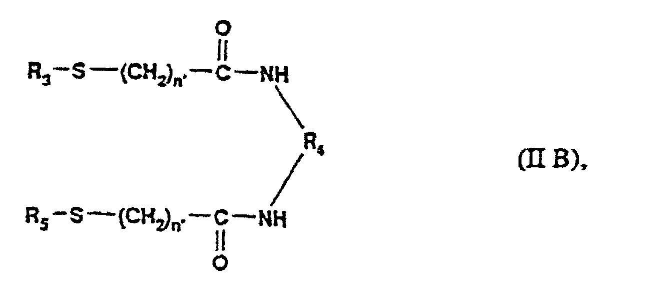
in which
R₂, R₃ and R₅ are, independently of one another, a hydrogen atom, or a (C₁₋₆-alkyl)CO-, (C₆₋₈-aryl)CO- or (C₇₋₉-arylalkyl)CO- radical which is optionally substituted by a hydroxyl group, a C₁₋₄-alkoxy, a carboxyl or a sulphonic acid radical, and
R₄ is a radical of the formula II C or II D 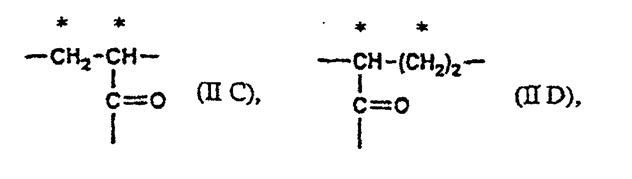 in which
the carbon atoms identified by an * are bonded to the imino groups of the formula II B, and
n' is the numbers 1 or 2,
i is any number from 2 to 6, and
TT is α- and/or β-amino acids which are connected in a conventional way via amide groups,
and chelating agent radicals derived from aminopolycarboxylic acids of the formula II K of II L 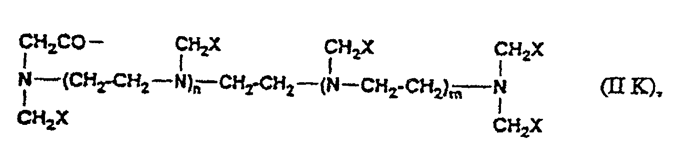 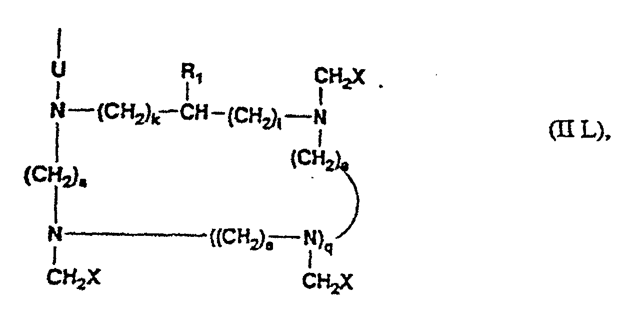 where
n and m are each the numbers 0, 1, 2, 3 or 4, where n and m do not total more than 4,
a is the numbers 2, 3, 4, or 5
k is the numbers 1, 2, 3, 4, or 5,
l is the numbers 0, 1, 2, 3, 4, or 5 and
q is the numbers 0, 1 or 2,
U is a hydrogen atom or a C₁₋₆-alkyl radical which is optionally substituted by one or more hydroxyl groups and contains a bond to L,
the X radicals are, independently of one another, each hydrogen atoms, COOH groups, ester groups or amide groups having 1-6 carbon atoms in the alkyl radical,
R₁ is a bond to L or a hydrogen atom,
and chelating agent radicals of the formula II M,
Cp(aa)Cp- (II M)
in which
Cp is a protected cysteine and (aa) is one of the naturally occurring amino acids,
and cysteine-rich amino acid sequences of the metallothioneins -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- and analogous sequences in which serine is replaced by threonine, glycine or alanine.

2. Compounds according to Claim 1, **characterized in that** the metal ion M is an ion of one of the isotopes of Tc, Re, In, Cr, Mn, Fe, Co, Ni, Cu, Pr, Nd, Sm, Y, Gd, Tb, Dy, Ho, Er and La.

3. Compounds according to at least one of Claims 1 to 2, **characterized in that** the endothelins E have the amino acid sequence Cys-Ser-Cys-Ser-Ser-Leu-Met-Asp-Lys-Glu-Cys-Val-Tyr-Phe-Cys-His-Leu-Asp-Ile-Ile-Trp or parts thereof.

4. Compounds according to at least one of Claims 1 to 2, **characterized in that** the parts of the endothelins E have the amino acid sequence of -His-Leu-Asp-Ile-Ile-Trp-.

5. Compounds according to at least one of Claims 1 to 2, **characterized in that** the endothelin analogues E have one of the amino acid sequences 
-Cys-Ser-Cys-Ser-Ser-Trp-Leu-Asp-Lys-Glu-Cys-Val-Tyr-Phe-Cys-His-Leu-Asp-Ile-
Ile-Trp-;
 
-Cys-Thr-Cys-Phe-Thr-Tyr-Lys-Asp-Lys-Glu-Cys-Val-Tyr-Tyr-Cys-His-Leu-Asp-
Ile-Ile-Trp-;
 
-Cys-Ser-Ala-Ser-Ser-Leu-Met-Asp-Lys-Glu-Ala-Val-Tyr-Phe-Cys-His-Leu-Asp-Ile-
Ile-Trp-;
 
-Cys-Ser-Cys-Asn-Ser-Trp-Leu-Asp-Lys-Glu-Cys-Val-Tyr-Phe-Cys-His-Leu-Asp-
Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-Asp-Ile-
Ile-Trp-;
 
-Ala-Ser-Ala-Ser-Ser-Leu-Met-Asp-Lys-Glu-Ala-Val-Tyr-Phe-Ala-His-Leu-Asp-Ile-
Ile-Trp-;
 
-Cys-Ser-Cys-Ser-Ser-Trp-Leu-Asp-Lys-Glu-Ala-Val-Tyr-Phe-Ala-His-Leu-Asp-Ile-
Ile-Trp-;
 or parts thereof.

6. Compounds according to at least one of Claims 1 to 2, **characterized in that** the endothelin antagonists E are one of the cyclic pentapeptides

-D Trp-D Asp-Pro-D Val-Leu

-D Glu-Ala-allo D Ile-Leu-D Trp-.

7. Compounds according to at least one of Claims 1 to 6, **characterized in that** the alkylene group standing for L is straight-chain, branched, cyclic, aliphatic, aromatic or arylaliphatic.

8. Compounds according to at least one of Claims 1 to 6, **characterized in that** L is Z₁-R-Z₂,
in which
Z₁ and Z₂ are, independently of one another, a group -(C=O)O-, -(C=O)-, -NH-, -NH-(C=O)-, and
R is a straight-chain mono- to decamethylene group, or a radical of the formula α 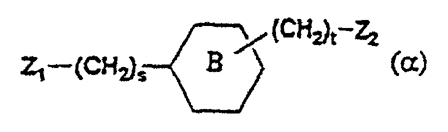 in which
s is equal to 1 and t is equal to 0, and phenylene for ring B, and
Z₁ and Z₂ are, independently of one another, a -NH-(C=S)-, -NH-(C=S)NH-, -(C=O)O-, -(C=O)-, -NH-, -NH-(C=O)- group.

9. Compounds according to at least one of Claims 1 to 8, **characterized in that** the complexing agent residue K is
an S-benzoylthioacetylglycylglycylglycine residue,
an N,N'-bis(benzoylthioacetyl)-2,3-diaminopropionic acid residue,
an N,N'-bis(benzoylthioacetyl)-3,4-diaminobutyric acid residue,
an N,N'-bis(benzoylthioacetyl)-4,5-diaminopentanoic acid residue,
a Cys(Acm)-Gly-Cys(Acm)-Gly-Gly-Arg-Gly-Asp-Ser residue,
an ethylenediaminetetraacetic acid residue,
a diethylenetriaminepentaacetic acid residue,
a trans-1,2-cyclohexanediaminetetraacetic acid residue,
a 1,4,7,10-tetraazacyclododecanetetraacetic acid residue,
a 1,4,7-triazacyclononanetriacetic acid residue,
a 1,4,8,11-tetraazatetradecanetetraacetic acid residue,
a 1,5,9-triazacyclododecanetriacetic acid residue,
a 1,4,7,10-tetraazacyclododecanetriacetic acid residue
or a 3,6,9,15-tetraazabicyclo[9,3,1]pentadeca-1(15),11,13-trienetriacetic acid residue.

10. Complexes of compounds of the general formula (I) with metal ions as defined in Claim 1, namely
a) ^{99m}Tc complex of S-benzoylthioacetyl-Gly-Gly-Gly-Gly-Asp-His-Leu-Asp-Ile-Ile-Trp,
b) 
99mTc- complex of N,N´-bis(S-benzoylthioacetyl)-3,4-diaminobutyryl-Cys-Ser-
Cys-Ser-Ser-Leu-Met-Asp-Lys-Glu-Cys-Val-Tyr-Phe-Cys-His-Leu-Asp-Ile-Ile-
Trp,
c) 
99mTc- complex of N,N´-bis(S-benzoylthioacetyl)-4,5-diamino-1-oxo-pentyl-
Cys-Ser-Cys-Ser-Ser-Leu-Met-Asp-Lys-Glu-Cys-Val-Tyr-Phe-Cys-His-Leu-Asp-
Ile-Ile-Trp,
d) ^{99m}Tc complex of N-[3,6,9-triaza-1-oxo-3,6,9,9-tetra(hydroxycarbonylmethyl)nonyl]-Gly-His-Leu-Asp-Ile-Ile-Trp,
e) ¹¹¹In complex of N-[3,6,9-triaza-1-oxo-3,6,9,9-tetra(hydroxycarbonylmethyl)nonyl]-Gly-His-Leu-Asp-Ile-Ile-Trp,
f) Gd(III) complex of N-[3,6,9-triaza-1-oxo-3,6,9,9-tetra(hydroxycarbonylmethyl)nonyl]-Gly-His-Leu-Asp-Ile-Ile-Trp, sodium salt,
g) Gd(III) complex of 1-{2-hydroxy-3-[4-(Gly-His-Leu-Asp-Ile-Ile-Trp-thiouridyl)phenoxy]propyl}-1,4,7,10-tetraaza-4,7,10-tris(carboxylatomethyl)cyclododecane,
h) 
99mTc- complex of Cys(Acm)-Gly-Cys(Acm)-Cys-Ser-Cys-Ser-Ser-Leu-Met-
Asp-Lys-Glu-Cys-Val-Tyr-Phe-Cys-His-Leu-Asp-Ile-Ile-Trp, ..
i) ^{99m}Tc-complex of cyclo(Trp-Leu-Val-Pro-Asp)-Cys(Acm)-Gly-Cys(Acm),
k) 
99mTc-complex of 4-mercaptobutyrimidyl-Cys-Ser-Ala-Ser-Ser-Leu-Met-Asp-
Lys-Glu-Ala-Val-Tyr-Phe-Cys-His-Leu-Asp-Ile-Ile-Trp.
l) ^{99m}Tc complex of 3-thiopropionyl-Gly-Asp-His-Leu-Asp-Ile-Ile-Trp,
m) ^{99m}Tc complex of 2-(acetylthio)succinyl-Gly-Asp-His-Leu-Asp-Ile-Ile-Trp.

11. Compounds of the general formula (I)
E-L-(K)_{b} (I)
in which
E is a radical derived from endothelins, endothelin derivatives, endothelin part-sequences, endothelin analogues or endothelin antagonists,
and where the radical E has the property of binding selectively to endothelin receptors,
L is a direct linkage or a Z₁-R-Z₂ radical,
in which
R is a straight-chain, branched, saturated or unsaturated C₁₋₂₀-alkyl radical which is optionally interrupted by one or more oxygen and/or sulphur atoms and/or carbonyl, -NHCO-, -N(C₁₋₆alkyl)CO-, -NH- and -N(C₁₋₆alkyl)-radicals and optionally substituted by hydroxyl and/or epoxy groups,
Z₁ and Z₂ are, independently of one another, a -O-, -S-, -(C=O)O-,-NH-(C=S)NH-, -(C=O)-, -(C=S)O-, -NH-, -NH-(C=O)- or -NH-(C=S)- group,
or a radical of the formula α 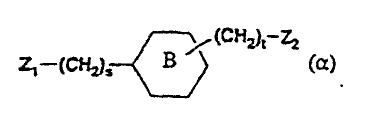 in which
s and t are independently of one another, the numbers 0, 1, 2 or 3, the ring B is a phenyl or cyclohexyl radical, and Z₁ and Z₂ have the meaning indicated above,
b is the number 1,
K is a chelating agent radical of the general formula II A or II B 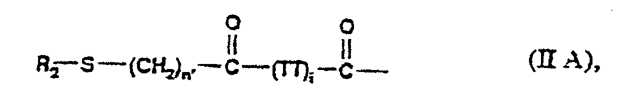 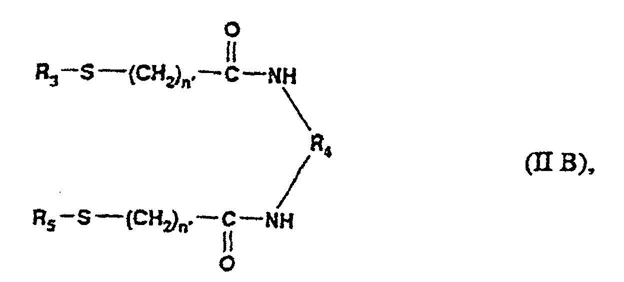
in which
R₂, R₃ and R₅ are, independently of one another, a hydrogen atom, or a (C₁₋₆-alkyl)CO-, (C₆₋₈-aryl)CO- or (C₇₋₉-arylalkyl)CO- radical which is optionally substituted by a hydroxyl group, a C₁₋₄-alkoxy, a carboxyl or a sulphonic acid radical, and
R₄ is a radical of the formula II C or II D 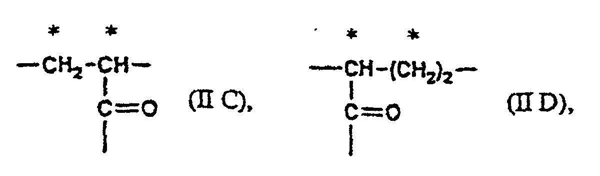 in which the carbon atoms identified by an * are bonded to the imino groups of the formula II B, and
n' is the numbers 1 or 2,
i is any number from 2 to 6, and
TT is α- and/or β-amino acids which are connected in a conventional way via amide groups,
and chelating agent radicals derived from aminopolycarboxylic acids of the formula II K or II L 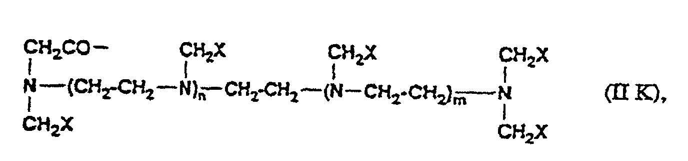 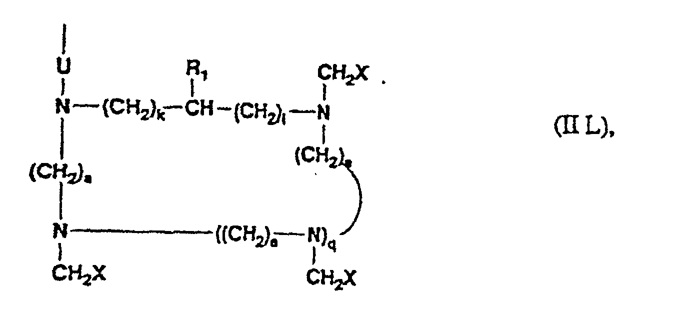 where
n and m are each the numbers 0, 1, 2, 3 or 4, where n and m do not total more than 4,
a is the numbers 2, 3, 4, or 5
k is the numbers 1, 2, 3, 4, or 5,
l is the numbers 0, 1, 2, 3, 4, or 5 and
q is the numbers 0, 1 or 2,
U is a hydrogen atom or a C₁₋₆-alkyl radical which is optionally substituted by one or more hydroxyl groups and contains a bond to L,
the X radicals are, independently of one another, each hydrogen atoms, COOH groups, ester groups or amide groups having 1-6 carbon atoms in the alkyl radical,
R₁ is a bond to L or a hydrogen atom,
and chelating agent radicals of the formula II M,
Cp(aa)Cp- (II M)
in which
Cp is a protected cysteine and (aa) is one of the naturally occurring amino acids,
and cysteine-rich amino acid sequences of the metallothioneins -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- and analogous sequences in which serine is replaced by threonine, glycine or alanine.

12. Compounds according to Claim 11, **characterized in that** the endothelins E have the amino acid sequence Cys-Ser-Cys-Ser-Ser-Leu-Met-Asp-Lys-Glu-Cys-Val-Tyr-Phe-Cys-His-Leu-Asp-Ile-Ile-Trp or parts thereof.

13. Compounds according to Claim 11, **characterized in that** the parts of the endothelins E have the amino acid sequence of -His-Leu-Asp-Ile-Ile-Trp-.

14. Compounds according to Claim 11, **characterized in that** the endothelin analogues E have one of the amino acid sequences 
-Cys-Ser-Cys-Ser-Ser-Trp-Leu-Asp-Lys-Glu-Cys-Val-Tyr-Phe-Cys-His-Leu-Asp-Ile-
Ile-Trp-;
 
-Cys-Thr-Cys-Phe-Thr-Tyr-Lys-Asp-Lys-Glu-Cys-Val-Tyr-Tyr-Cys-His-Leu-Asp-
Ile-Ile-Trp-;
 
-Cys-Ser-Ala-Ser-Ser-Leu-Met-Asp-Lys-Glu-Ala-Val-Tyr-Phe-Cys-His-Leu-Asp-Ile-
Ile-Trp-;
 
-Cys-Ser-Cys-Asn-Ser-Trp-Leu-Asp-Lys-Glu-Cys-Val-Tyr-Phe-Cys-His-Leu-Asp-
Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-Asp-Ile-
Ile-Trp-;
 
-Ala-Ser-Ala-Ser-Ser-Leu-Met-Asp-Lys-Glu-Ala-Val-Tyr-Phe-Ala-His-Leu-Asp-Ile-
Ile-Trp-;
 
-Cys-Ser-Cys-Ser-Ser-Trp-Leu-Asp-Lys-Glu-Ala-Val-Tyr-Phe-Ala-His-Leu-Asp-Ile-
Ile-Trp-;
 or parts thereof.

15. Compounds according to Claim 11, **characterized in that** the endothelin antagonists E are one of the cyclic pentapeptides -D Trp-D Asp-Pro-D Val-Leu or

-D Glu-Ala-allo D Ile-Leu-D Trp-.

16. Compounds according to at least one of Claims 11 to 15, **characterized in that** the alkylene group standing for L is straight-chain, branched, cyclic, aliphatic, aromatic or arylaliphatic.

17. Compounds according to at least one of Claims 11 to 15, **characterized in that** L is Z₁-R-Z₂,
in which
Z₁ and Z₂ are, independently of one another, a group -(C=O)O-, -(CO)-, -NH-, -NH-(C=O)-, and
R is a straight-chain mono- to decamethylene group, or a radical of the formula α 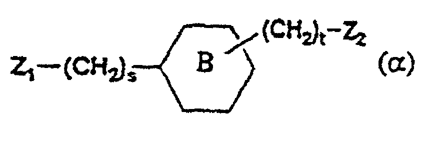 in which
s is equal to 1 and t is equal to 0, and phenylene for ring B, and
Z₁ and Z₂ are, independently of one another, a -NH-(C=S)-, -NH-(C=S)NH-, -(CO)O-, -(C=O)-, -NH-, -NH-(C=O)- group.

18. Compounds according to at least one of Claims 11 to 17, **characterized in that** the complexing agent residue K is
an S-benzoylthioacetylglycylglycylglycine residue,
an N,N'-bis(benzoylthioacetyl)-2,3-diaminopropionic acid residue,
an N,N'-bis(benzoylthioacetyl)-3,4-diaminobutyric acid residue,
an N,N'-bis(benzoylthioacetyl)-4,5-diaminopentanoic acid residue,
a Cys(Acm)-Gly-Cys(Acm)-Gly-Gly-Arg-Gly-Asp-Ser residue,
an ethylenediaminetetraacetic acid residue,
a diethylenetriaminepentaacetic acid residue,
a trans-1,2-cyclohexanediaminetetraacetic acid residue,
a 1,4,7,10-tetraazacyclododecanetetraacetic acid residue,
a 1,4,7-triazacyclononanetriacetic acid residue,
a 1,4,8,11-tetraazatetradecanetetraacetic acid residue,
a 1,5,9-triazacyclododecanetriacetic acid residue,
a 1,4,7,10-tetraazacyclododecanetriacetic acid residue
or a 3,6,9,15-tetraazabicyclo[9,3,1]pentadeca-1(15),11,13-trienetriacetic acid residue.

19. Process for preparing complexes of compounds of the general formula (I) with metal ions as defined in Claim 1, **characterized in that** a radioactive metal ion is reacted in the form of its permetallate in a manner known per se, in the presence of a reducing agent and, where appropriate, of an auxiliary ligand, with a compound of the general formula (I) according to Claim 1
E-L-(K)_{b} (I).

20. Process for preparing complexes of compounds of the general formula (I) with metal ions as defined in Claim 1, **characterized in that** a suitable salt or oxide of a suitable paramagnetic and/or of a radioactive cation is reacted in a manner known per se with a compound of the general formula (I) according to Claim 1
E-L-(K)_{b} (I).

21. Process according to Claim 19, **characterized in that** technetium-99m or Re is employed in the form of pertechnetate or perrhenate.

22. Process according to Claim 20, **characterized in that** ¹¹¹In is employed as radioactive cation.

23. Process according to Claim 20, **characterized in that** Gd is employed as paramagnetic cation.

24. Process for preparing a compound of the general formula (I), **characterized in that** E', which is an endothelin, an endothelin derivative, an endothelin part-sequence, an endothelin analogue or an endothelin antagonist, is reacted in a manner known per se with a compound of the formula (III)
(K)_{b}-L-H (III)
where K, L and b have the meaning stated in Claim 1.

25. Process according to Claim 24, **characterized in that** the linkage between E and L is in the form of an ester, ether, thioether, thioester, amide or thioamide linkage.

26. Cold kit for preparing radiopharaceuticals, consisting of a compound of the general formula (I) according to any of Claims 11 to 18, a reducing agent and, where appropriate, an auxiliary ligand, which are present in the dry state or in solution, instructions for use with a reaction method for the conversion of the described compound with technetium-99m or Re in the form of a pertechnetate solution or perrhenate solution.

27. Use of a compound according to any of Claims 1 to 10 for producing compositions for the diagnosis of vascular disorders.

28. Use of a compound according to any of Claims 11 to 18 for producing compositions for the diagnosis of vascular disorders using metal ions M according to Claim 2.

## Revendications

1. Complexes de composés de formule générale (I)
E-L-(K)_{b} (I)
avec des ions métalliques de nombres atomiques 21-32, 37-39, 42-51 et 57-83,
dans laquelle
E représente un radical, dérivé d'endothélines, de dérivés d'endothéline, de séquences partielles d'endothéline, d'analogues d'endothéline ou d'antagonistes d'endothéline, le radical E présentant la propriété de se fixer sélectivement sur les récepteurs d'endothéline,
L représente une liaison directe ou un radical Z₁-R-Z₂,
dans lequel
R représente un radical alkyle en C₁ à C₂₀ saturé ou insaturé, linéaire, ramifié, le cas échéant interrompu par un ou plusieurs atomes d'oxygène et/ou de soufre et/ou radicaux carbonyle, -NHCO-, -N(alkyle en C₁ à C₆)CO-, -NH- et -N(alkyle en C₁ à C₆) et le cas échéant substitué par des groupements hydroxy et/ou époxy,
Z₁ et Z₂ sont, indépendamment l'un de l'autre, un groupement -O-, -S-, -(C=O)O-, -NH-(C=S)NH-, -(C=O)-, -(C=S)O-, -NH-, -NH-(C=O)- ou -NH-(C=S)-
ou L représente un radical de formule α 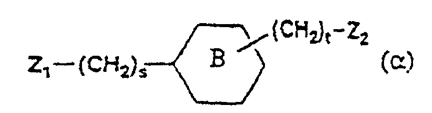 dans laquelle s et t représentent indépendamment l'un de l'autre les chiffres 0, 1, 2, ou 3, le cycle B signifie un radical phényle ou cyclohexyle et Z₁ et Z₂ présentent la signification indiquée précédemment,
b représente le chiffre 1,
K représente un radical d'un chélatant de formule générale II A ou II B 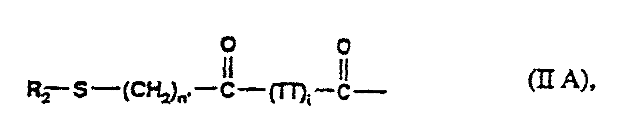 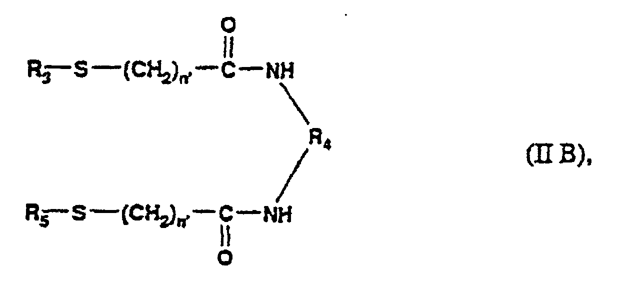 dans lesquelles
R₂, R₃ et R₅ représentent indépendamment l'un de l'autre un atome d'hydrogène, un radical (alkyle en C₁ à C₆)CO-, un radical (aryle en C₆ à C₈)CO- ou un radical (arylalkyle en C₇ à C₉)CO-, le cas échéant substitués par un groupement hydroxyle, un radical alcoxy en C₁ à C₄, un radical carboxyle ou un radical acide sulfonique et
R₄ représente un radical de formule II C ou II D 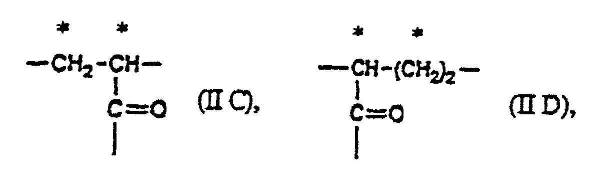 dans lesquelles
les atomes de carbone **caractérisés par** * sont liés aux groupements imino de la formule II B et
n' représente les chiffres 1 ou 2,
i représente un nombre quelconque de 2 à 6, et
TT représente des acides α-aminés et/ou β-aminés, qui sont reliés de manière usuelle via des groupements amide,
ainsi que des radicaux de chélatants, qui sont dérivés des acides aminopolycarboxyliques de formules II K ou II L 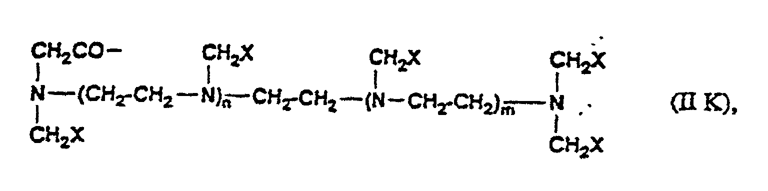 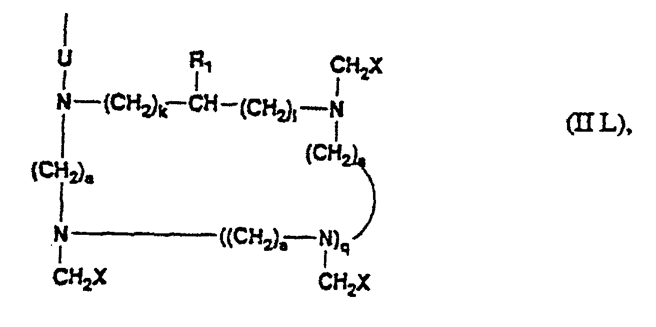 où
n et m représentent à chaque fois les chiffres 0, 1, 2, 3 ou 4, n et m n'étant pas supérieurs à 4,
a représente les chiffres 2, 3, 4 ou 5,
k représente les chiffres 1, 2, 3, 4 ou 5,
l représente les chiffres 0, 1, 2, 3, 4 ou 5 et
q représente les chiffres 0, 1 ou 2,
U représente un atome d'hydrogène ou un radical alkyle en C₁ à C₆ qui est le cas échéant substitué par un ou plusieurs groupements hydroxy et qui contient une liaison avec L,
les radicaux X signifient indépendamment l'un de l'autre à chaque fois des atomes d'hydrogène, des groupements COOH, des groupements ester ou des groupements amide comprenant 1 à 6 atomes de carbone dans le radical alkyle,
R₁ représente une liaison avec L ou un atome d'hydrogène,
ainsi que des radicaux de chélatants de formule II M
Cp(aa)Cp- (II M)
dans laquelle Cp représente de la cystéine protégée et (aa) un des acides aminés existant à l'état naturel ainsi que des séquences d'acides aminés riches en cystéine des métallothionines -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- et des séquences analogues, dans lesquelles la sérine est remplacée par de la thréonine, de la glycine ou de l'alanine.

2. Composés selon la revendication 1, **caractérisés en ce que** l'ion métallique M est un ion d'un des isotopes du Tc, Re, In, Cr, Mn, Fe, Co, Ni, Cu, Pr, Nd, Sm, Y, Gd, Tb, Dy, Ho, Er et La.

3. Composés selon au moins l'une quelconque des revendications 1 à 2, **caractérisés en ce que** les endothélines E présentent la séquence d'acides aminés Cys-Ser-Cys-Ser-Ser-Leu-Met-Asp-Lys-Glu-Cys-Val-Tyr-Phe-Cys-His-Leu-Asp-Ile-Ile-Trp ou des parties de celle-ci.

4. Composés selon au moins l'une quelconque des revendications 1 à 2, **caractérisés en ce que** les parties des endothélines E présentent la séquence d'acides aminés -His-Leu-Asp-Ile-Ile-Trp-.

5. Composés selon au moins l'une quelconque des revendications 1 à 2, **caractérisés en ce que** les analogues de l'endothéline E présentent une des séquences d'acides aminés 
-Cys-Ser-Cys-Ser-Ser-Trp-Leu-Asp-Lys-Glu-Cys-Val-Tyr-Phe-Cys-His-Leu-Asp-Ile-
Ile-Trp-;
 
-Cys-Thr-Cys-Phe-Thr-Tyr-Lys-Asp-Lys-Glu-Cys-Val-Tyr-Tyr-Cys-His-Leu-Asp-
Ile-Ile-Trp-;
 
-Cys-Ser-Ala-Ser-Ser-Leu-Met-Asp-Lys-Glu-Ala-Val-Tyr-Phe-Cys-His-Leu-Asp-Ile-
Ile-Trp-;
 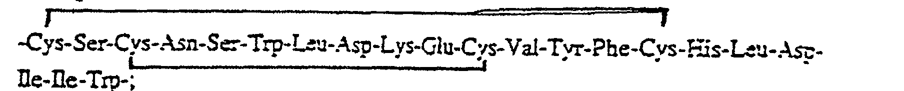
-Cys-Ser-Cys-Asn-Ser-Trp-Leu-Asp-Lys-Glu-Cys-Val-Tyr-Phe-Cys-His-Leu-Asp-
Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-Asp-Ile-
Ile-Trp-;
 
-Ala-Ser-Ala-Ser-Ser-Leu-Met-Asp-Lys-Glu-Ala-Val-Tyr-Phe-Ala-His-Leu-Asp-Ile-
Ile-Trp-;
 
-Cys-Ser-Cys-Ser-Ser-Trp-Leu-Asp-Lys-Glu-Ala-Val-Tyr-Phe-Ala-His-Leu-Asp-Ile-
Ile-Trp-;
 ou des parties de celles-ci.

6. Composés selon au moins l'une quelconque des revendications 1 à 2, **caractérisés en ce que** les antagonistes de l'endothélin E sont un des pentapeptides cycliques

-D Trp-D Asp-Pro-D Val-Leu-

-D Glu-Ala-allo D Ile-Leu-D Trp-.

7. Composés selon au moins l'une quelconque des revendications 1 à 6, **caractérisés en ce que** le groupement alkylène représenté par L est linéaire, ramifié, cyclique, aliphatique, aromatique ou arylaliphatique.

8. Composés selon au moins l'une quelconque des revendications 1 à 6, **caractérisés en ce que** L représente Z₁-R-Z₂
où
Z₁ et Z₂ signifient indépendamment l'un de l'autre un groupement -(C=O)O-, -(C=O)-, -NH-, -NH-(C=O)- et
R représente un groupement linéaire monométhylène à décaméthylène
ou un radical de formule α 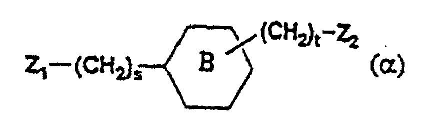 dans laquelle
s est égal à 1 et t à 0 et le cycle B représente du phénylène et
Z₁ et Z₂ représentent, indépendamment l'un de l'autre, un groupement -NH-(C=S)-, -NH-(C=S)NH-, -(C=O)O-, -(C=O)-, -NH-, -NH-(C=O)-.

9. Composés selon au moins l'une quelconque des revendications 1 à 8, **caractérisés en ce que** le radical de complexant K représente un radical S-benzoylthioacétylglycylglycylglycine, un radical acide N,N'-bis(benzoylthioacétyl)-2,3-diaminopropionique, un radical acide N,N'-bis(benzoylthioacétyl)-3,4-diaminobulyrique, un radical acide N,N'-bis(benzoylthioacétyl)-4,5-diaminopentanoïque, un radical Cys(Acm)-Gly-Cys(Acm)-Gly-Gly-Arg-Gly-Asp-Ser, un radical acide éthylènediaminetétraacétique, un radical acide diéthylènetriaminepentaacétique, un radical acide trans-1,2-cyclohexanediaminetétraacétique, un radical acide 1,4,7,10-tétraazacyclododécanetétraacétique, un radical acide 1,4,7-triazacyclononanetriacétique, un radical acide 1,4,8,11-tétraazatétradécanetétraacétique, un radical acide 1,5,9-triazacyclododécanetriacétique, un radical acide 1,4,7,10-tétraazacyclododécanetriacétique ou un radical acide 3,6,9,15-tétraazabicyclo[9,3,15]pentadécal(15),11,13-triènetriacétique.

10. Complexes de composés de formule générale (I) avec des ions métalliques selon la définition dans la revendication 1, à savoir,
a) complexe ^{99m}Tc de S-benzoylthioacétyl-Gly-Gly-Gly-Gly-Asp-His-Leu-Asp-Ile-Ile-Trp,
b) 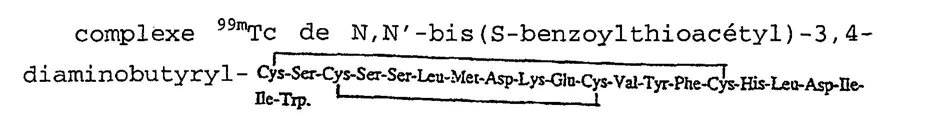
complexe 99mTc de N,N'-bis(S-benzoylthioacétyl)-3,4-
diaminobutyryl- Cys-Ser-Cys-Ser-Ser-Leu-Met-Asp-Lys-Glu-Cys-Val-Tyr-Phe-Cys-His-Leu-Asp-Ile-
Ile-Trp.
c) 
complexe ^{99m}Tc de N,N'-bis(S-benzoylthioacétyl)-4,5-
diamino-1-oxo-pentyl-
Cys-Ser-Cys-Ser-Ser-Leu-Met-Asp-Lys-Glu-Cys-Val-Tyr-Phe-Cys-His-Leu-Asp-
Ile-Ile-Trp,
d) complexe ^{99m}Tc de N-[3,6,9-triaza-1-oxo-3,6,9,9-tétra-(hydroxycarbonylméthyl)-nonyl]-Gly-His-Leu-Asp-Ile-Ile-Trp,
e) complexe ¹¹¹In de N-[3,6,9-triaza-1-oxo-3,6,9,9-tétra-(hydroxycarbonylméthyl)-nonyl]-Gly-His-Leu-Asp-Ile-Ile-Trp,
f) sel sodique du complexe Gd(III) de N-[3,6,9-triaza-1-oxo-3,6,9,9-tétra-(hydroxycarbonylméthyl)-nonyl]-Gly-His-Leu-Asp-Ile-Ile-Trp,
g) complexe Gd(III) de 1-{2-hydroxy-3-[4-(Gly-His-Leu-Asp-Ile-Ile-Trp-thiouridyl)phénoxy]propyl}1,4,7,10-tétraaza-4,7,10-tris(carboxylatométhyl)cyclododécane,
h) 
complexe ⁹⁹ᵐTc de Cys(Acm)-Gly-Cys(Acm)-Cys-Ser-Cys-Ser-Ser-Leu-Met-
Asp-Lys-Glu-Cys-Val-Tyr-Phe-Cys-His-Leu-Asp-Ile-Ile-Trp,
i) complexe ^{99m}Tc de cyclo(Trp-Leu-Val-Pro-Asp)-Cys(Acm)-Gly-Cys(Acm),
k) 
complexe ^{99m}Tc de 4-mercaptobutyrimidyl-
-Cys-Ser-Ala-Ser-Ser-Leu-Met-Asp- Lys-Glu-Ala-Val-Tyr-Phe-Cys-His-Leu-Asp-Ile-Ile-Trp,
l) complexe ^{99m}Tc de 3-thiopropionyl-Gly-Asp-His-Leu-Asp-Ile-Ile-Trp,
m) complexe ^{99m}Tc de 2-(acétylthio)succinyl-Gly-Asp-His-Leu-Asp-Ile-Ile-Trp.

11. Composés de formule générale (I)
E-L-(K)_{b} (I)
dans laquelle
E représente un radical dérivé d'endothélines, de dérivés d'endothéline, de séquences partielles d'endothéline, d'analogues d'endothéline ou d'antagonistes d'endothéline, le radical E présentant
la propriété de se fixer sélectivement sur les récepteurs d'endothéline,
L représente une liaison directe ou un radical Z₁-R-Z₂, dans lequel
R représente un radical alkyle en C₁ à C₂₀ saturé ou insaturé, linéaire, ramifié, le cas échéant interrompu par un ou plusieurs atomes d'oxygène et/ou de soufre et/ou radicaux carbonyle, -NHCO-, -N(alkyle en C₁ à C₆)CO-, -NH- et -N(alkyle en C₁ à C₆) et le cas échéant substitué par des groupements hydroxy et/ou époxy,
Z₁ et Z₂ sont, indépendamment l'un de l'autre, un groupement -O-, -S-, -(C=O)O-, -NH-(C=S)NH-, -(C=O)-, -(C=S)O-, -NH-, -NH-(C=O)- ou -NH-(C=S)-
ou L représente un radical de formule α 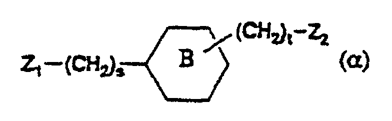 dans laquelle s et t représentent indépendamment l'un de l'autre les chiffres 0, 1, 2, ou 3, le cycle B signifie un radical phényle ou cyclohexyle et Z₁ et Z₂ présentent la signification indiquée précédemment,
b représente le chiffre 1,
K représente un radical d'un chélatant de formule générale II A ou II B 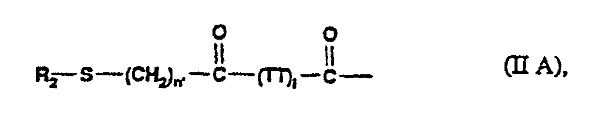 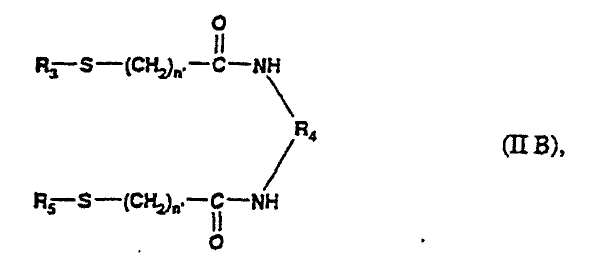 dans lesquelles
R₂, R₃ et R₅ représentent indépendamment l'un de l'autre un atome d'hydrogène, un radical (alkyle en C₁ à C₆)CO-, un radical (aryle en C₆ à C₈)CO- ou un radical (arylalkyle en C₇ à C₉)CO-, le cas échéant substitués par un groupement hydroxyle, un radical alcoxy en C₁ à C₄, un radical carboxyle ou un radical acide sulfonique et
R₄ représente un radical de formule II C ou II D 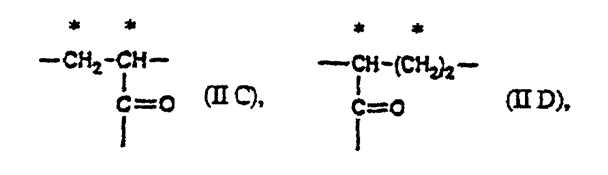 dans lesquelles
les atomes de carbone **caractérisés par** * sont liés aux groupements imino de la formule II B et
n' représente les chiffres 1 ou 2,
i représente un nombre quelconque de 2 à 6, et
TT représente des acides α-aminés et/ou β-aminés, qui sont reliés de manière usuelle via des groupements amide,
ainsi que des radicaux de chélatants, qui sont dérivés des acides aminopolycarboxyliques de formules II K ou II L 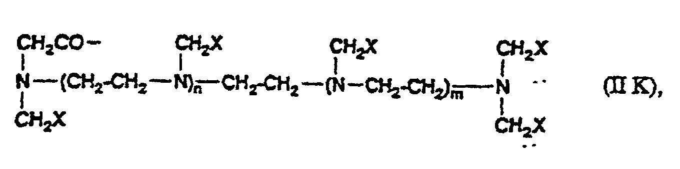 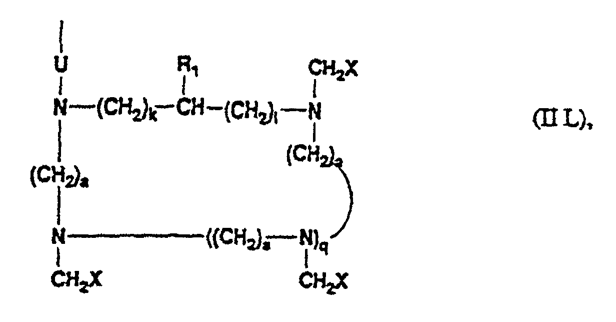 où
n et m représentent à chaque fois les chiffres 0, 1, 2, 3 ou 4, n et m n'étant pas supérieurs à 4,
a représente les chiffres 2, 3, 4 ou 5,
k représente les chiffres 1, 2, 3, 4 ou 5,
1 représente les chiffres 0, 1, 2, 3, 4 ou 5 et
q représente les chiffres 0, 1 ou 2,
U représente un atome d'hydrogène ou un radical alkyle en C₁ à C₆ qui est le cas échéant substitué par un ou plusieurs groupements hydroxy et qui contient une liaison avec L,
les radicaux X signifient indépendamment l'un de l'autre à chaque fois des atomes d'hydrogène, des groupements COOH, des groupements ester ou des groupements amide comprenant 1 à 6 atomes de carbone dans le radical alkyle,
R₁ représente une liaison avec L ou un atome d'hydrogène,
ainsi que des radicaux de chélatants de formule II M
Cp(aa)Cp- (II M)
dans laquelle Cp représente de la cystéine protégée et (aa) un des acides aminés existant à l'état naturel ainsi que des séquences d'acides amines riches en cystéine des métallothionines -Ser-Cys-Thr-Cys-Thr-Ser-Ser-Cys-Ala-, -Ala-Cys-Lys-Ala-Cys-Lys-Cys-, -Gly-Cys-Ser-Lys-Cys-Ala-Gln-Gly-Cys-Val-, -Cys-Lys-Gly-Ala-Ala-Asp-Lys-Cys-Thr-Cys-Cys-Ala- et des séquences analogues, dans lesquelles la sérine est remplacée par de la thréonine, de la glycine ou de l'alanine.

12. Composés selon la revendication 11, **caractérisés en ce que** les endothélines E présentent la séquence d'acides aminés Cys-Ser-Cys-Ser-Ser-Leu-Met-Asp-Lys-Glu-Cys-Val-Tyr-Phe-Cys-His-Leu-Asp-Ile-Ile-Trp ou des parties de celle-ci.

13. Composés selon la revendication 11, **caractérisés en ce que** les parties des endothélines E présentent la séquence d'acides aminés -His-Leu-Asp-Ile-Ile-Trp-.

14. Composés selon la revendication 11, **caractérisés en ce que** les analogues de l'endothéline E présentent une des séquences d'acides aminés 
-Cys-Ser-Cys-Ser-Ser-Trp-Leu-Asp-Lys-Glu-Cys-Val-Tyr-Phe-Cys-His-Leu-Asp-Ile-
Ile-Trp-;
 
-Cys-Thr-Cys-Phe-Thr-Tyr-Lys-Asp-Lys-Glu-Cys-Val-Tyr-Tyr-Cys-His-Leu-Asp-
Ile-Ile-Trp-;
 
-Cys-Ser-Ala-Ser-Ser-Leu-Met-Asp-Lys-Glu-Ala-Val-Tyr-Phe-Cys-His-Leu-Asp-Ile-
Ile-Trp-;
 
-Cys-Ser-Cys-Asn-Ser-Trp-Leu-Asp-Lys-Glu-Cys-Val-Tyr-Phe-Cys-His-Leu-Asp-
Ile-Ile-Trp-;
 
-Cys-Ser-Cys-Lys-Asp-Met-Thr-Asp-Lys-Glu-Cys-Leu-Asn-Phe-Cys-His-Gln-Asp-
Val-Ile-Trp-;
 
-Ala-Ser-Cys-Ser-Ser-Leu-Met-Asp-Lys-Glu-Cys-Val-Tyr-Phe-Ala-His-Leu-Asp-Ile-
Ile-Trp-:
 
-Ala-Ser-Ala-Ser-Ser-Leu-Met-Asp-Lys-Glu-Ala-Val-Tyr-Phe-Ala-His-Leu-Asp-Ile-
Ile-Trp-;
 
-Cys-Ser-Cys-Ser-Ser-Trp-Leu-Asp-Lys-Glu-Ala-Val-Tyr-Phe-Ala-His-Leu-Asp-Ile-
Ile-Trp-;
 ou des parties de celles-ci.

15. Composés selon la revendication 11, **caractérisés en ce que** les antagonistes de l'endothéline E sont un des pentapeptides cycliques

-D Trp-D Asp-Pro-D Val-Leu-

-D Glu-Ala-allo D Ile-Leu-D Trp-.

16. Composés selon au moins l'une quelconque des revendications 11 à 15, **caractérisés en ce que** le groupement alkylène représenté par L est linéaire, ramifié, cyclique, aliphatique, aromatique ou arylaliphatique.

17. Composés selon au moins l'une quelconque des revendications 11 à 15, **caractérisés en ce que** L représente Z₁-R-Z₂
où
Z₁ et Z₂ signifient indépendamment l'un de l'autre un groupement -(C=O)O-, -(C=O)-, -NH-, -NH-(C=O)- et
R représente un groupement linéaire monométhylène à décaméthylène
ou un radical de formule α 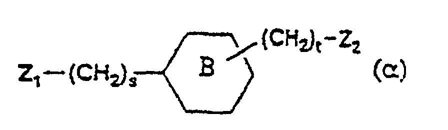 dans laquelle
s est égal à 1 et t à 0 et le cycle B représente du phénylène et
Z₁ et Z₂ représentent, indépendamment l'un de l'autre, un groupement -NH-(C=S)-, -NH-(C=S)NH-, -(C=O)O-, -(C=O)-, -NH-, -NH-(C=O)-.

18. Composés selon au moins l'une quelconque des revendications 11 à 17, **caractérisés en ce que** le radical de complexant K représente un radical S-benzoylthioacétylglycylglycylglycine, un radical acide N,N'-bis(benzoylthioacétyl)-2,3-diaminopropionique, un radical acide N,N'-bis(benzoylthioacétyl)-3,4-diaminobutyrique, un radical acide N,N'-bis(benzoylthioacétyl)-4,5-diaminopentanoïque, un radical Cys(Acm)-Gly-Cys(Acm)-Gly-Gly-Arg-Gly-Asp-Ser, un radical acide éthylènediaminetétraacétique, un radical acide diéthylènetriaminepentaacétique, un radical acide trans-1,2-cyclohexanediaminetétraacétique, un radical acide 1,4,7,10-tétraazacyclododécanetétraacétique, un radical acide 1,4,7-triazacyclononanetriacétique, un radical acide 1,4,8,11-tétraazatétradécanetétraacétique, un radical acide 1,5,9-triazacyclododécanetriacétique, un radical acide 1,4,7,10-tétraazacyclododécanetriacétique ou un radical acide 3,6,9,15-tétraazabicyclo[9,3,15]pentadécal(15),11,13-triènetriacétique.

19. Procédé pour la préparation de complexes de composés de formule générale (I) avec des ions métalliques, tels que définis dans la revendication 1,
**caractérisé en ce qu'**on transforme de manière connue en soi un ion métallique radioactif sous forme de son permétallate, en présence d'un réducteur et le cas échéant d'un ligand auxiliaire avec un composé de formule générale (I) selon la revendication 1
E-L-(K)_{b} (I).

20. Procédé pour la préparation des complexes de composés de formule générale (I) avec des ions métalliques, tels que définis dans la revendication 1, **caractérisé en ce qu'**on transforme de manière connue en soi un sel ou un oxyde approprié d'un cation paramagnétique et/ou radioactif approprié avec un composé de formule générale (I) selon la revendication 1
E-L-(K)_{b} (I).

21. Procédé selon la revendication 19, **caractérisé en ce qu'**on utilise du technétium-99m ou du Re sous forme de pertechnétate ou de perrhénate.

22. Procédé selon la revendication 20, **caractérisé en ce qu'**on utilise comme cation radioactif du ¹¹¹In.

23. Procédé selon la revendication 20, **caractérisé en ce qu'**on utilise comme cation paramagnétique du Gd.

24. Procédé pour la préparation d'un composé de formule générale (I), **caractérisé en ce qu'**on transforme de manière connue en soi E', qui représente une endothéline, un dérivé d'endothéline, une séquence partielle d'endothéline, un analogue d'endothéline ou un antagoniste d'endothéline avec un composé de formule (III)
(K)_{b}-L-H (III),
où K, L et b ont la signification indiquée dans la revendication 1.

25. Procédé selon la revendication 24, **caractérisé en ce que** la liaison entre E et L se présente sous forme d'une liaison ester, éther, thioéther, thioester, amide ou thioamide.

26. Kit de marquage à froid pour la préparation de produits radiopharmaceutiques constitué d'un composé de formule générale (I) selon l'une quelconque des revendications 11 à 18, d'un réducteur et le cas échéant d'un ligand auxiliaire, qui se trouvent sous forme sèche ou en solution, un mode d'emploi avec une prescription de réaction pour la transformation du composé décrit avec du technétium-99m ou du Re sous forme d'une solution de pertechnétate ou de perrhénate.

27. Utilisation d'un composé selon l'une quelconque des revendications 1 à 10 pour la préparation d'agents pour le diagnostic de maladies cardio-vasculaires.

28. Utilisation d'un composé selon l'une quelconque des revendications 11 à 18 pour la préparation d'agents pour le diagnostic de maladies cardio-vasculaires en utilisant des ions métalliques M selon la revendication 2.
